# EUROPÄISCHE PATENTSCHRIFT

(11) **EP 3 015 083 B1**
(45) Veröffentlichungstag und Bekanntmachung des Hinweises auf die Patenterteilung: **18.05.2022**
(21) Anmeldenummer: 15190707.8
(22) Anmeldetag: 21.10.2015
(51) Int. Cl.: A61B 17/34

(54) **DICHTEINRICHTUNG ZUR ABDICHTUNG EINER DURCHFÜHRUNG FÜR EIN MEDIZINISCHES INSTRUMENT**
SEALING DEVICE FOR SEALING A FEEDTHROUGH FOR A MEDICAL INSTRUMENT
DISPOSITIF D'ETANCHEITE DESTINE A ETANCHEIFIER UN PASSAGE POUR UN INSTRUMENT MEDICAL

(30) Priorität: 03.11.2014 DE 102014115985
(43) Veröffentlichungstag der Anmeldung: 04.05.2016
(73) Patentinhaber: Karl Storz SE & Co. KG, 78532 Tuttlingen (DE)
(72) Erfinder: Wagner, Sebastian, 78532 Tuttlingen (DE); Teufel, Felix, 78532 Tuttlingen (DE); Müller, Annika, 78532 Tuttlingen (DE)

(56) Entgegenhaltungen:
- EP-A2- 2 016 972
- WO-A1-99/42152
- WO-A1-2005/092217
- WO-A1-2007/121425
- US-A- 5 584 850
- US-A- 5 752 938
- US-A- 5 779 697
- US-A- 6 123 689
- US-A1- 2008 065 111
- US-B1- 6 811 546

## Beschreibung

Die vorliegende Erfindung ist auf eine Dichteinrichtung zur Abdichtung einer Durchführung für ein medizinisches Instrument, insbesondere zur Abdichtung des Zwischenraums zwischen einem Tubus und einem während eines mikroinvasiven medizinischen Eingriffs in dem Tubus angeordneten Schafts eines medizinischen Instruments, gerichtet.

Ein Beispiel für mikroinvasive medizinische Methoden ist die Laparoskopie. Mittels eines Trokars wird ein künstlicher Zugang zur Bauchhöhle eines Patienten durch dessen Bauchdecke hindurch geschaffen. Durch das Lumen eines während des laparoskopischen Eingriffs in der Bauchdecke verbleibenden Tubus des Trokars können ein Endoskop und/oder weitere medizinische Instrumente (beispielsweise Zangen, Scheren, Nadelhalter) in die Bauchhöhle eingeführt werden. Während der Laparoskopie wird der Bauchraum mit Kohlenstoffdioxid oder einem anderen Gas gefüllt, um ein Pneumoperitoneum, einen den medizinischen Eingriff ermöglichenden Hohlraum, zu schaffen. Ohne besondere Maßnahmen würde dieses Gas durch den Tubus entweichen. Deshalb wurden zahlreiche Ansätze entwickelt, um das Lumen des Tubus eines Trokars möglichst fluiddicht zu verschließen, und zwar sowohl im leeren Zustand als auch bei eingeführtem Instrument.

Teilweise ähnliche Aufgabestellungen können sich an einem Arbeitskanal eines Endoskops oder beim Einführen eines Katheters in ein Blutgefäß ergeben. Im letztgenannten Fall ist jedoch nicht das Entweichen eines Gases, sondern von Blut zu verhindern.

In US 4,857,062 ist ein Ventil zum Einführen eines Katheters in eine Arterie beschrieben. Zur Abdichtung ist ein flexibles Element vorgesehen, das zur Ausbildung einer fluiddichten Abdichtung mit einem Katheter komprimiert ist.

In WO 93/01850 A1 ist eine durch Hebel betätigte Dichtung für einen Tubus beschrieben. Eine Wand aus einem Elastomer mit einer Öffnung wird beim Einführen eines Instruments in den Tubus durch mehrere Hebel gedehnt, wodurch die Öffnung vergrößert wird.

In US 5,366,446 ist eine Einführanordnung zur Verwendung auf der Haut eines Patienten beschrieben, die für das Einführen von Tuben mit unterschiedlichen Außendurchmessern ausgebildet ist. Die Anordnung umfasst eine Membran aus einem durchstechbaren elastomeren Material in der Mitte eines Faltenbalgs.

In EP 0 746 359 B1 ist ein Katheter-Sperrventil beschrieben. Zur Abdichtung sind eine Gummidichtung mit einer Öffnung und distal dieser ein Entenschnabelventil mit einem geraden Schlitz vorgesehen.

In US 4,430,081 ist eine Kanüle zur Verwendung mit Angiographiekathetern beschrieben. Zur Abdichtung gegen das Eindringen von Luft oder das Austreten von Blut aus einem Blutgefäß sind eine erste Dichtung mit einem Schlitz, eine zweite Dichtung mit einem Loch und eine dritte Dichtung mit einer Klappe darin vorgesehen, die aneinander angrenzend angeordnet sind.

In WO 91/12838 A1 ist ein Infusionszugang mit mehreren hintereinander angeordneten elastischen Scheiben, die jeweils kreisförmige Öffnungen oder gegeneinander verdreht angeordnete sternförmige Schlitze aufweisen, vorgesehen.

In WO 98/32484 A1 ist ein Kathetereinführungsbesteck mit einem Hämostaseventil beschrieben. Ein Dichtelement umfasst zwei gelochte Stützscheiben, zwischen denen eine Dichtscheibe aus weich-elastischem Schaumkunststoff mit radial verlaufenden Schlitzen vorgesehen ist.

In US 6,551,282 B1 ist eine Dichtung zur Abdichtung eines Tubus bei eingeführtem Schaft eines Endoskops beschrieben. Eine breite ringförmige Dichtung ist von einem steiferen Ring umgeben.

In US 6,123,689 ist eine wiederverwendbare Trokar-Kanüle mit einer Einweg-Dichtung beschrieben. Die Einweg-Dichtung weist eine oder mehrere Dichtlippen auf.

In US 2008/0065111 A1 ist eine Dichtung an einer Kanüle beschrieben (Absätze [0083] bis [0085]; Figuren 18A, 18B, 19).

In WO 2007/121425 A1 ist eine Trokar-Kanüle mit einer Instrumentendichtung beschrieben. Die Instrumentendichtung weist radiale Falten auf (Figuren 3, 4, 5). In den Figuren 4 und 5 ist der kreisförmige innere Rand der Instrumentendichtung erkennbar.

In EP 2 016 972 A2 ist eine Dichtung für eine Trokar-Kanüle beschrieben. Der äußere Rand einer Dichtlippe ist polygonal, ihr innerer Rand ist kreisförmig.

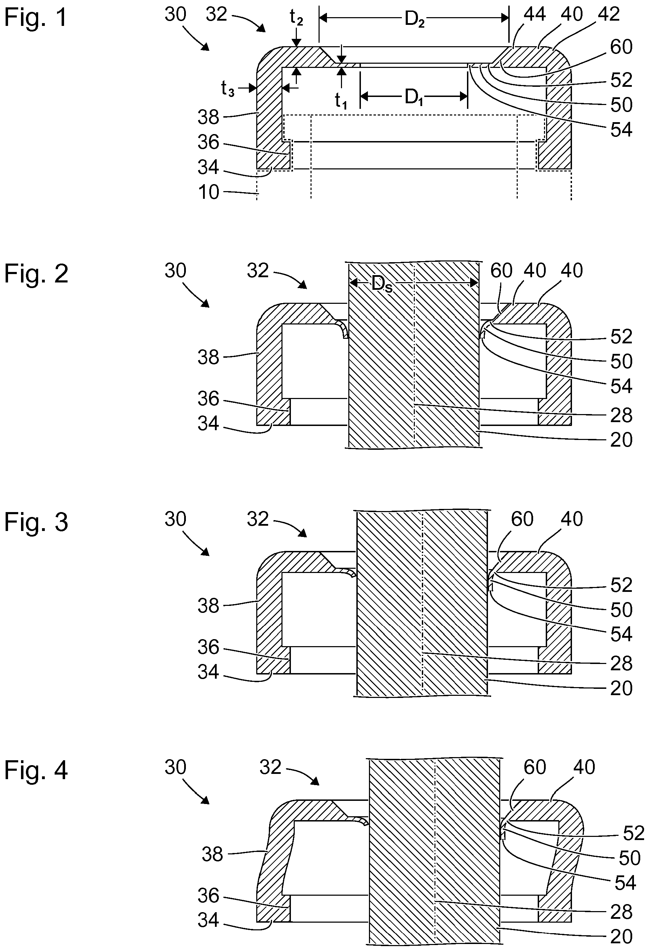

In US 5,779,697 ist eine Kanüle für die Arthroskopie mit einer Fluiddichtung beschrieben. Die Dichtung weist eine kreisförmige Öffnung 142 auf (Spalte 6, Zeile 61, Figuren 2, 3, 15).

In US 6,811,546 B1 ist eine Dichtung für einen mikroinvasiv-chirurgischen Zugang beschrieben.

In WO 2005/092217 A1 ist eine Dichtung für eine Trokarkanüle beschrieben.

In US 5,752,938 ist eine Dichtung für ein chirurgisches Instrument beschrieben. Eine Membran umfasst einen oder mehrere Lappen, zwischen denen ein chirurgisches Instrument eingeführt werden kann. Das chirurgische Instrument 500 ist in der Dichtung radial bewegbar, wobei die Dichtung verformt wird.

Die beschriebenen Dichteinrichtungen weisen jeweils spezifische Vor- und Nachteile auf. Eine sehr weitgehende und zuverlässige Dichtwirkung ist ohne Weiteres mit einer Dichtlippe erzielbar, die mit einem möglichst hohen Anpressdruck gegen die äußere Oberfläche eines Schafts eines Instruments drückt. Dabei entsteht jedoch hohe Gleit- und Haftreibung. Eine dünne und/oder weiche Dichtlippe erzeugt nur geringe Gleit- und Haftreibung, ist aber nicht nur weniger robust, sondern weist bei einer seitlichen Verschiebung des Schafts in der Dichteinrichtung eine reduzierte Wirkung auf.

Bei allen bekannten dünnen und weichen Dichtlippen ist bei einer Änderung der Richtung der Bewegung eines Schafts in der Dichteinrichtung ein Umlegen der Dichtlippe zu beobachten, das mit einer fühlbaren Änderung der zwischen Schaft und Dichteinrichtung wirkenden Kraft einher geht. Diese Änderung der Kraft kann eine unwillkürliche oder dem Willen des medizinischen Personals nicht genau entsprechende Bewegung des Schafts bewirken, die als störend empfunden wird oder sogar den Patienten gefährden kann.

Eine Aufgabe der vorliegenden Erfindung besteht darin, eine verbesserte Dichteinrichtung zu schaffen, die insbesondere die aufgezählten Anforderungen und Erwartungen in ausgewogenen Verhältnissen erfüllt.

Diese Aufgabe wird durch die Gegenstände der unabhängigen Ansprüche gelöst.

Weiterbildungen sind in den abhängigen Ansprüchen angegeben.

Viele herkömmliche Dichteinrichtungen weisen möglichst breite Dichtlippen auf, um einer Bewegung eines Schafts eines Instruments in der Dichteinrichtung in einer Richtung orthogonal zur Längsachse des Schafts einen geringen Widerstand entgegenzusetzen, und um eine Verwendbarkeit mit Schäften mit unterschiedlichen Durchmessern zu ermöglichen. Ferner beruhte die Verwendung breiter Dichtlippen auf der Annahme, dass diese aufgrund ihrer großen Elastizität einer axialen Bewegung eines Schafts relativ zur Dichteinrichtung nur einen geringen Widerstand entgegensetzt. Die vorliegende Erfindung beruht auf der Erkenntnis, dass die breiten Dichtlippen weniger eine Lösung bieten als ein Problem schaffen. Breite Dichtlippen müssen eine bestimmte Mindestwandstärke aufweisen, um beispielsweise durch den Überdruck im Pneumoperitoneum nicht aufgeblasen oder verformt zu werden. Eine Verwendbarkeit für viele verschiedene Schaftdurchmesser hat dann zwangsläufig eine große mechanische Spannung bei einem großen Schaftdurchmesser zur Folge, die wiederum die Reibung erhöht.

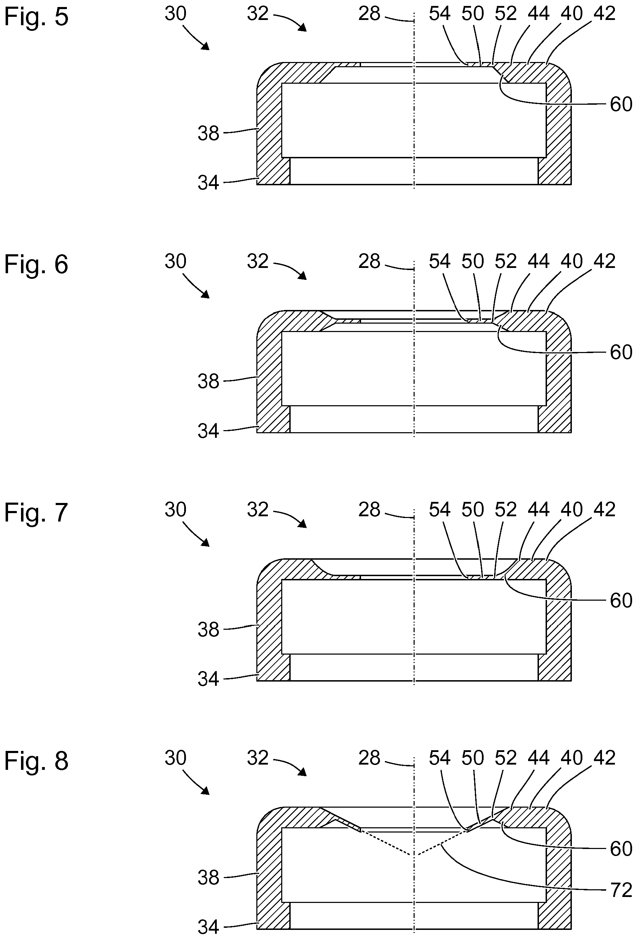

Im Übrigen ist eine Bewegbarkeit eines Schafts relativ zur Dichteinrichtung in einer Richtung orthogonal zur Längsachse des Schafts nicht nur durch breite und elastische Dichtlippen erzielbar. In der bereits erwähnten US 6,551,282 B1 ist zwar den Figuren 4 und 5 entnehmbar, dass die genannte Bewegbarkeit auch durch andere Teile der Dichteinrichtung gewährleistet werden kann. Ähnliches gilt für US 7,803,135 B2. Es ist jedoch bei beiden Druckschriften nicht erkennbar, ob die genannte Erkenntnis vorlag. Jedenfalls sind die Autoren der US 6,551,282 B1 vom Grundansatz breiter Dichtlippen nicht abgewichen. Die Autoren der US 7,803,135 B2 haben erkennbar einen Weg beschritten, der vom nachfolgend beschriebenen Lösungsansatz weg führt.

Ausführungsformen der vorliegenden Erfindung beruhen auf der Idee, eine Verschiebbarkeit eines Schafts in einer Dichteinrichtung in einer Richtung orthogonal zur Längsachse des Schafts nicht mittels der Dichtlippe zu ermöglichen, sondern die Dichtlippe vergleichsweise schmal auszugestalten. Die genannte Verschiebbarkeit muss dann zwar durch andere Maßnahmen gewährleistet sein, die Dichtlippe kann aber ohne die Gefahr einer Verformung durch eine Druckdifferenz vergleichsweise dünn ausgeführt werden. Durch einen die Dichtlippe umgebenden Stützbereich mit vergleichsweise geringer Elastizität einerseits und eine präzise Anpassung der Dichtlippe an einen vorbestimmten Schaftdurchmesser können eine zuverlässige Dichtwirkung und gleichzeitig geringe Reibung und eine nur geringe Kraftvariation beim Umlegen der Dichtlippe nach Richtungsänderung erzielt werden.

Ausführungsformen der vorliegenden Erfindung beruhen ferner auf der Erkenntnis, dass das Umlegen der Dichtlippe nach Richtungsänderung der Bewegung eines Schafts in der Dichteinrichtung dann besonders spürbar und störend ist, wenn es entlang des gesamten Umfangs der Dichtlippe gleichzeitig stattfindet, und dass dies durch die übliche rotationssymmetrische Ausgestaltung von Dichteinrichtungen und ihren Dichtlippen begünstigt wird. Ausführungsformen der vorliegenden Erfindung beruhen deshalb auf der Idee, die Rotationssymmetrie der Dichtlippe zu brechen, um ein nicht gleichzeitiges, sondern allmähliches Umlegen der Dichtlippe - ausgehend von einem Punkt und sich entlang ihres Umfangs ausbreitend - zu begünstigen.

Eine Dichteinrichtung zur Abdichtung einer Durchführung für einen Schaft eines medizinischen Instruments umfasst einen Befestigungsbereich zur Befestigung der Dichteinrichtung an einem Tubus oder einer anderen medizinischen Vorrichtung, einen ringartigen Stützbereich, der mit dem Befestigungsbereich verbunden ist, und eine ringartige Dichtlippe mit einem äußeren Rand, der mit einem inneren Rand des Stützbereichs verbunden ist, und einem inneren Rand, der vorgesehen und ausgebildet ist, um an einer äußeren Oberfläche eines in die Dichteinrichtung eingeführten Schafts fluiddicht anzuliegen, wobei die Differenz U₂ - U_{S} des Umfangs U₂ des inneren Rands des Stützbereichs und des Umfangs U_{S} eines Schafts eines medizinischen Instruments, für den die Dichteinrichtung vorgesehen und ausgebildet ist, nicht größer ist als die Differenz U_{S} - U₁ des Umfangs U_{S} des Schafts und des Umfangs U₁ des inneren Rands der Dichtlippe in ihrem spannungsfreien Zustand (U₂ - U_{S} ≤ U_{S} - U₁) oder die Differenz D₂ - D_{S} des mittleren Durchmessers D₂ des inneren Rands des Stützbereichs und des mittleren Durchmessers D_{S} eines Schafts eines medizinischen Instruments, für den die Dichteinrichtung vorgesehen und ausgebildet ist, nicht größer ist als die Differenz D_{S} - D₁ des mittleren Durchmessers D_{S} des Schafts und des mittleren Durchmessers D₁ des inneren Rands der Dichtlippe D₂ - D_{S} ≤ D_{S} - D₁.

Die genannte Bedingung für die Umfänge kann alternativ so formuliert werden, dass der arithmetische Mittelwert (U₁ + U₂)/2 des Umfangs U₁ des inneren Rands der Dichtlippe und des Umfangs U₂ des inneren Rands des Stützbereichs nicht größer ist als der Umfang U_{S} eines Schafts eines medizinischen Instruments, für den die Dichteinrichtung vorgesehen und ausgebildet ist ((U₁ + U₂)/2 ≤ D_{S}). Die genannte Bedingung für die mittleren Durchmesser kann alternativ so formuliert werden dass der arithmetische Mittelwert (D₁ + D₂)/2 des mittleren Durchmessers D₁ des inneren Rands der Dichtlippe und des mittleren Durchmessers D₂ des inneren Rands des Stützbereichs nicht größer ist als der mittlere Durchmesser D_{S} eines Schafts eines medizinischen Instruments, für den die Dichteinrichtung vorgesehen und ausgebildet ist.

Eine Dichteinrichtung zur Abdichtung einer Durchführung für einen Schaft eines medizinischen Instruments umfasst einen Befestigungsbereich zur Befestigung der Dichteinrichtung an einem Tubus oder einer anderen medizinischen Vorrichtung, einen ringartigen Stützbereich, der mit dem Befestigungsbereich verbunden ist, und eine ringartige Dichtlippe mit einem äußeren Rand, der mit einem inneren Rand des Stützbereichs verbunden ist, und einem inneren Rand, der vorgesehen und ausgebildet ist, um an einer äußeren Oberfläche eines in die Dichteinrichtung eingeführten Schafts fluiddicht anzuliegen, wobei der Umfang des inneren Rands der Dichtlippe mindestens sechs Zehntel und höchstens neun Zehntel des Umfangs eines Schafts eines medizinischen Instruments, für den die Dichteinrichtung vorgesehen und ausgebildet ist, beträgt

Eine Dichteinrichtung zur Abdichtung einer Durchführung für einen Schaft eines medizinischen Instruments umfasst einen Befestigungsbereich zur Befestigung der Dichteinrichtung an einem Tubus oder einer anderen medizinischen Vorrichtung, einen ringartigen Stützbereich, der mit dem Befestigungsbereich verbunden ist, und eine ringartige Dichtlippe mit einem äußeren Rand, der mit einem inneren Rand des Stützbereichs verbunden ist, und einem inneren Rand, der vorgesehen und ausgebildet ist, um an einer äußeren Oberfläche eines in die Dichteinrichtung eingeführten Schafts fluiddicht anzuliegen, wobei die mittlere Dicke der Dichtlippe insbesondere höchstens ein Fünftel der mittleren Dicke des Stützbereichs beträgt.

Bei den hier beschriebenen Dichteinrichtungen ist die Dichtlippe jeweils insbesondere membranartig. Insbesondere weist die Dichtlippe eine konstante Dicke oder eine in radialer Richtung und/oder in umfänglicher Richtung jeweils nur geringfügig (insbesondere um einen Faktor zwei oder weniger) variierende Dicke auf.

Soweit die inneren Ränder des Stützbereichs und der Dichtlippe und die äußere Kontur des Querschnitts des Schafts eines medizinischen Instruments, für den die Dichteinrichtung vorgesehen und ausgebildet ist, jeweils kreisförmig sind, sind die mittleren Durchmesser die Durchmesser und die Bedingungen für die Umfänge und die Durchmesser äquivalent. Als mittlerer Durchmesser eines Rands, der nur in Abschnitten, die zusammen weniger als die Hälfte oder weniger als ein Drittel des Umfangs des Rands ausmachen, von der Gestalt eines Kreises abweicht, wird der Durchmesser des Kreises angesehen. Als mittlerer Durchmesser eines anderen nicht kreisförmigen Rands wird der Durchmesser eines Kreises, dessen Flächeninhalt dem Inhalt der von dem Rand begrenzten Fläche entspricht, angesehen.

Wenn der Stützbereich und die Dichtlippe das gleiche Material aufweisen und einstückig ausgebildet sind und zwischen dem Stützbereich und der Dichtlippe keine stufenförmige Änderung der Dicke vorliegt, ist der innere Rand des Stützbereichs die (linienförmige) Menge der Orte, an denen die Kraft, die die Dichteinrichtung einer Verformung durch einen Schaft eines medizinischen Instruments, für den die Dichteinrichtung vorgesehen und ausgebildet ist, entgegensetzt, stark ansteigt. Der innere Rand des Stützbereichs ist beispielsweise die (linienförmige) Menge der Orte, an denen die Kraft, die die Dichteinrichtung einer Verformung durch einen Schaft eines medizinischen Instruments, für den die Dichteinrichtung vorgesehen und ausgebildet ist, entgegensetzt, gegenüber dem minimalen Wert verdoppelt oder verdreifacht oder verfünffacht oder verzehnfacht ist. Der innere Rand des Stützbereichs ist insbesondere die Linie, an der die Dicke der Dichteinrichtung dem arithmetischen Mittelwert der minimalen Dicke der Dichtlippe und der maximalen Dicke des Stützbereichs oder 90 % der Dicke des Stützbereichs entspricht. Wenn der Stützbereich die Gestalt eines ringartigen Ausschnitts einer Platte mit konstanter Dicke aufweist und kontinuierlich in die Dichtlippe übergeht, ist der innere Rand des Stützbereichs insbesondere der innere Rand des plattenförmigen Bereichs konstanter Dicke. Der äußere Rand des Stützbereichs liegt beispielsweise dort, wo der Stützbereich in eine im Wesentlichen zylindrische Wand oder unmittelbar in einen Befestigungsbereich übergeht.

Die Dichteinrichtung ist insbesondere zur Abdichtung des ringförmigen oder ringartigen Zwischenraums zwischen der inneren Oberfläche eines Tubus eines Trokars und der äußeren Oberfläche eines im Tubus angeordneten Schafts eines medizinischen Instruments vorgesehen. Das medizinische Instrument ist beispielsweise ein Endoskop oder ein anderes medizinisches Instrument zur Verwendung bei mikroinvasiven medizinischen Maßnahmen. Die Dichteinrichtung ist für ein medizinisches Instrument mit einem Schaft mit einem vorbestimmten Querschnitt, insbesondere mit einem kreisförmigen Querschnitt mit einem vorbestimmten Durchmesser, vorgesehen und ausgebildet. Um eine Verwechslung oder eine unbeabsichtigte Verwendung für einen Schaftquerschnitt, für den die Dichteinrichtung nicht vorgesehen und ausgebildet ist, zu vermeiden, kann die Dichteinrichtung mit einer alphanumerischen Beschriftung, einem Symbol oder einer Farbe eindeutig gekennzeichnet sein. Insbesondere kann der Schaftdurchmesser, für den die Dichteinrichtung vorgesehen und ausgebildet ist, an der Dichteinrichtung alphanumerisch angegeben sein.

Der Stützbereich und die Dichtlippe können das gleiche Material aufweisen, insbesondere ist die gesamte Dichteinrichtung einstückig ausgebildet, beispielsweise als Gussteil, das den Befestigungsbereich, den Stützbereich und die Dichtlippe umfasst. Alternativ können die Dichtlippe einerseits und der Stützbereich und/oder der Befestigungsbereich andererseits unterschiedliche Materialien aufweisen, wobei Bereiche aus unterschiedlichen Materialien insbesondere stoff- und/oder formschlüssig miteinander verbunden sind. Beispielsweise sind die Dichtlippe aus einem vergleichsweise weichen Material und der Stützbereich oder ein Teil des Stützbereichs oder die zylindrische Wand 38 aus einem vergleichsweise harten Material gebildet.

Der Befestigungsbereich umfasst beispielsweise einen nach innen ragenden Kragen am distalen bzw. dem Patienten und dem Tubus zuzuwendenden Rand, der eine formschlüssige mechanische Verbindung mit einer Nut am proximalen bzw. der Dichteinrichtung zuzuwendenden Rand eines Tubus oder einer anderen medizinischen Vorrichtung bilden kann.

Die Dichteinrichtung ist insbesondere teilweise oder vollständig aus einem Silikonelastomer, einem anderen Elastomer oder einem anderen elastischen Material gebildet. Das Material der Dichteinrichtung oder zumindest das Material der Dichtlippe weist insbesondere eine Härte von ca. 40 Shore A auf. Die Elastizität des Materials, insbesondere im Befestigungsbereich kann das Bilden und das Lösen einer formschlüssigen mechanischen Verbindung der Dichteinrichtung mit einem Tubus oder einer anderen medizinischen Vorrichtung ermöglichen.

Ein Stützbereich oder eine Dichtlippe ist ringartig, wenn der Stützbereich bzw. die Dichtlippe die Topologie eines Kreisrings aufweist, also im mathematischen Sinne zweifach zusammenhängend ist. Weder der ringartige Stützbereich noch die ringartige Dichtlippe muss kreisringförmig sein. Die inneren und äußeren Ränder des Stützbereichs und der Dichtlippe können jeweils kreisförmig, elliptisch, oval, polygonal sein oder eine andere Gestalt aufweisen.

Eine Dichteinrichtung mit den beschriebenen Merkmalen kann eine sehr schmale Dichtlippe, also eine Dichtlippe, bei der die Differenz der Durchmesser oder die Differenz der Umfänge des äußeren und des inneren Rands klein ist, aufweisen, ohne bei einer seitlichen Verschiebung eines in die Dichteinrichtung eingeführten Schafts eine Aufhebung der Dichtwirkung zu bewirken. Eine schmale Dichtlippe kann wiederum besonders dünnwandig und besonders elastisch ausgebildet sein, ohne die Dichtwirkung zu beeinträchtigen. Eine dünnwandige und elastische Ausgestaltung der Dichtlippe kann die Reibung zwischen der Dichtlippe und einem Schaft reduzieren und die Auswirkung des Umlegens der Dichtlippe bei Umkehrung der Bewegungsrichtung eines Schafts in der Dichtrichtung minimieren.

Bei einer Dichteinrichtung, wie sie hier beschrieben ist, ist die Dichtlippe insbesondere nicht rotationssymmetrisch zur Längsachse eines in der vorgesehenen mittleren Position und Orientierung in die Dichteinrichtung eingeführten Schafts.

Eine Dichteinrichtung zur Abdichtung einer Durchführung für einen Schaft eines medizinischen Instruments umfasst einen Befestigungsbereich zur Befestigung der Dichteinrichtung an einem Tubus oder einer anderen medizinischen Vorrichtung und eine ringartige Dichtlippe mit einem inneren Rand, der vorgesehen und ausgebildet ist, um an einer äußeren Oberfläche eines in die Dichteinrichtung eingeführten Schafts fluiddicht anzuliegen, wobei die Dichtlippe nicht rotationssymmetrisch zur Längsachse eines in der vorgesehenen mittleren Position und Orientierung in die Dichteinrichtung eingeführten Schafts ist.

Die Längsachse eines Schafts ist im Fall eines Schafts mit gerader und kreiszylindrischer Oberfläche die Symmetrieachse dieser Oberfläche. Die vorgesehene mittlere Position und Orientierung eines Schafts in der Dichteinrichtung ergibt sich aus der Geometrie der Dichteinrichtung. Beispielsweise ist der distale Rand und/oder die Befestigungseinrichtung nahe dem distalen Rand der Dichteinrichtung rotationssymmetrisch zur Längsachse eines in der vorgesehenen mittleren Position und Orientierung in die Dichteinrichtung eingeführten Schafts. Ferner sind die Position und die Orientierung der Längsachse eines in der vorgesehenen mittleren Position und Orientierung in die Dichteinrichtung eingeführten Schafts in der Regel durch den Tubus bzw. die medizinische Vorrichtung (insbesondere dessen bzw. deren Symmetrieachse) vorbestimmt, für den bzw. die die Dichteinrichtung vorgesehen und ausgebildet ist.

Alternativ dazu, dass die Dichtlippe nicht rotationssymmetrisch zur Längsachse eines in der vorgesehenen mittleren Position und Orientierung in die Dichteinrichtung eingeführten Schafts ist, kann die Dichtlippe nicht rotationssymmetrisch zur Längsachse eines in die Dichteinrichtung eingeführten Schafts beim mechanischen spannungsärmsten Zustand der Dichteinrichtung sein.

Eine nicht rotationssymmetrische Ausgestaltung der Dichtlippe kann zur Folge haben, dass in der Dichtlippe vorliegende Kräfte und mechanische Spannungen und zwischen der Dichtlippe (insbesondere ihrem inneren Rand) und einer äußeren Oberfläche eines Schafts wirkende Kräfte entlang des inneren Rands der Dichtlippe variieren. Dies wiederum kann bei einer Umkehr der Richtung, in der ein Schaft in der Dichteinrichtung bewegt wird, ein allmähliches, von einem Ort am inneren Rand der Dichtlippe ausgehendes und sich von dort vergleichsweise langsam ausbreitendes Umlegen der Dichtlippe bewirken. Die Wirkung des Umlegens, das von Anwendern herkömmlicher Dichteinrichtungen auch als Umschnappen bezeichnet wird, kann damit deutlich reduziert werden. Dies kann ein feinfühligeres Bewegen eines Schafts eines medizinischen Instruments in der Dichteinrichtung ermöglichen und das Risiko einer unwillkürlichen oder dem Willen des medizinischen Personals nicht genau entsprechenden Bewegung eines Schafts in der Dichteinrichtung und einer resultierenden Schädigung eines Patienten reduzieren.

Eine Dichteinrichtung, wie sie hier beschrieben ist, umfasst insbesondere ferner einen ringartigen Stützbereich, der mit dem Befestigungsbereich verbunden ist, wobei ein äußerer Rand der Dichtlippe mit dem inneren Rand des Stützbereichs verbunden ist.

Bei einer Dichteinrichtung, wie sie hier beschrieben ist, ist insbesondere zumindest entweder der innere Rand oder der äußere Rand der Dichtlippe nicht kreisförmig.

Bei einer Dichteinrichtung, wie sie hier beschrieben ist, ist insbesondere der Flächenmittelpunkt der durch den inneren Rand der Dichtlippe begrenzten Fläche vom Flächenmittelpunkt der durch den äußeren Rand der Dichtlippe begrenzten Fläche beabstandet.

Bei einer Dichteinrichtung, wie sie hier beschrieben ist, weist die Dichtlippe insbesondere im mechanisch spannungsfreien Zustand eine in Richtung ihres Umfangs variierende Dicke auf.

Bei einer Dichteinrichtung, wie sie hier beschrieben ist, sind insbesondere die Flächennormalen zweier voneinander abgewandter Oberflächen der Dichtlippe an einander gegenüberliegenden Orten an den Oberflächen im mechanisch spannungsfreien Zustand der Dichtlippe nicht parallel.

Die Flächennormalen zweier voneinander abgewandter Oberflächen der Dichtlippe an einander gegenüberliegenden Orten an den Oberflächen schließen insbesondere einen Winkel im Bereich von 1 Grad bis 3 Grad oder im Bereich von 1,5 Grad bis 2 Grad ein. Die beschriebene Nicht-Parallelität der Flächennormalen liegt insbesondere für die gesamte Fläche der Dichtlippe oder für mindestens die Hälfte der Fläche der Dichtlippe vor.

Bei einer Dichteinrichtung, wie sie hier beschrieben ist, weist der innere Rand der Dichtlippe insbesondere die Gestalt eines Ausschnitts einer Mantelfläche eines Zylinders, dessen Symmetrieachse nicht parallel zur mittleren Flächennormalen zweier voneinander abgewandter Oberflächen der Dichtlippe ist, auf.

Der innere Rand der Dichtlippe weist insbesondere die Gestalt eines Ausschnitts einer Mantelfläche eines schiefen Kreiszylinders oder eines anderen Zylinders auf. Die Symmetrieachse, d.h. die Richtung der Translationsinvarianz der Mantelfläche des Zylinders, schließt mit der mittleren Flächennormale zweier voneinander abgewandter Oberflächen insbesondere einen Winkel von mindestens 5 Grad oder mindestens 10 Grad oder mindestens 20 Grad oder mindestens 30 Grad ein. Die mittlere Flächennormale ist insbesondere der Mittelwert oder die halbe Differenz der normierten Integrale der Flächennormalen über die beiden voneinander abgewandten Oberflächen der Dichtlippe.

Bei einer Dichteinrichtung, wie sie hier beschrieben ist, ist der innere Rand der Dichtlippe insbesondere abgerundet, beispielsweise mit einem kreisbogenförmigen Querschnitt.

Bei einer Dichteinrichtung, wie sie hier beschrieben ist, weist die Dichtlippe insbesondere eine vom inneren Rand der Dichtlippe nach außen kontinuierlich ansteigende Dicke auf.

Bei einer Dichteinrichtung, wie sie hier beschrieben ist, ist die mittlere Flächennormale der Dichtlippe insbesondere nicht parallel zur Längsachse eines in der vorgesehenen mittleren Position und Orientierung in die Dichteinrichtung eingeführten Schafts.

Die mittlere Flächennormale der Dichtlippe ist insbesondere das normierte Integral der Flächennormalen von einer von zwei voneinander abgewandten Oberflächen der Dichtlippe über die gesamte Dichtlippe. Alternativ ist die mittlere Flächennormale beispielsweise der Mittelwert oder die halbe Differenz der normierten Integrale der Flächennormalen über die beiden voneinander abgewandten Oberflächen der Dichtlippe. Der Winkel zwischen der mittleren Flächennormalen und der Längsachse eines in der vorgesehenen mittleren Position und Orientierung in die Dichteinrichtung eingeführten Schafts beträgt insbesondere mindestens 5 Grad oder mindestens 10 Grad oder mindestens 20 Grad oder mindestens 30 Grad.

Bei einer Dichteinrichtung, wie sie hier beschrieben ist, ist der Stützbereich insbesondere parallel oder im Wesentlichen parallel zu einer Ebene, die nicht parallel zur Längsachse eines in der vorgesehenen mittleren Position und Orientierung in die Dichteinrichtung eingeführten Schafts ist.

Der gesamte Stützbereich oder ein proximaler Oberflächenbereich und/oder ein distaler Oberflächenbereich des Stützbereichs kann jeweils im Wesentlichen die Gestalt eines - insbesondere ringförmigen - Ausschnitts einer Ebene aufweisen, deren Normale nicht parallel zur Längsachse eines in der vorgesehenen mittleren Position und Orientierung in die Dichteinrichtung eingeführten Schafts ist.

Ferner können der gesamte Stützbereich oder ein proximaler Oberflächenbereich und/oder ein distaler Oberflächenbereich des Stützbereichs kann jeweils die Gestalt eines - insbesondere ringförmigen - Ausschnitts einer gekrümmten Flächen aufweisen, beispielsweise einer Kugeloberfläche oder einer Oberfläche eines anderen Rotationsellipsoids.

Jede der beschriebenen (und miteinander kombinierbaren) Arten einer nicht rotationssymmetrischen Ausgestaltung der Dichtlippe kann eine Variation der Kräfte zwischen der Dichtlippe und der Oberfläche eines eingeführten Schafts und/oder der mechanischen Spannung innerhalb der Dichtlippe bewirken, die ein allmähliches statt eines gleichzeitigen Umlegens der Dichtlippe nach Änderung der Bewegungsrichtung eines Schafts in der Dichteinrichtung zur Folge haben kann. Die Dichtlippe ist dabei immer vorgesehen und ausgebildet, um - anders als bei einer Dichtung mit einem oder mehreren radialen Schnitten - entlang einer umlaufenden Linie an der äußeren Oberfläche eines eingeführten Schafts anzuliegen und somit eine vollständige oder sehr weitgehende Dichtwirkung zu entfalten.

Bei einer Dichteinrichtung, wie sie hier beschrieben ist, beträgt der Umfang des inneren Rands der Dichtlippe mindestens sechs Zehntel und höchstens neun Zehntel des Umfangs eines Schafts eines medizinischen Instruments, für den die Dichteinrichtung vorgesehen und ausgebildet ist.

Bei einer Dichteinrichtung, wie sie hier beschrieben ist, ist der innere Rand der Dichtlippe insbesondere im Wesentlichen kreisförmig, und der Durchmesser des inneren Rands der Dichtlippe beträgt mindestens sechs Zehntel und höchstens neun Zehntel des Durchmessers eines kreiszylindrischen Schafts eines medizinischen Instruments, für den die Dichteinrichtung vorgesehen und ausgebildet ist.

Der Umfang bzw. der Durchmesser des inneren Rands der Dichtlippe kann alternativ mindestens sieben Zehntel und/oder höchstens acht Zehntel des Umfangs bzw. des Durchmessers eines Schafts, für den die Dichteinrichtung vorgesehen und ausgebildet ist, betragen.

Wenn die Gestalt des inneren Rands der Dichtlippe nur abschnittsweise von einer idealen Kreisform abweicht, ist mit dem Durchmesser des inneren Rands der Dichtlippe insbesondere der Durchmesser des Kreises gemeint.

Ein Verhältnis der Umfänge oder Durchmesser des inneren Rands der Dichtlippe und der äußeren Kontur des Querschnitts eines Schafts, für den die Dichteinrichtung vorgesehen und ausgebildet ist, im Bereich von sechs oder sieben Zehntel bis zu acht oder neun Zehntel kann ein besonders günstiger Kompromiss zwischen guter und zuverlässiger Dichtwirkung einerseits und geringer Reibung zwischen Schaft und Dichteinrichtung andererseits ermöglichen.

Bei einer Dichteinrichtung, wie sie hier beschrieben ist, beträgt die mittlere Dicke der Dichtlippe insbesondere höchstens ein Fünftel der mittleren Dicke des Stützbereichs.

Die Dicke der Dichtlippe beträgt insbesondere ca. 0,3 mm. Ein Verhältnis der Dicke der Dichtlippe zur Dicke des Stützbereichs von höchstens 1:5 schafft einerseits eine elastische Dichtlippe und eine geringe Reibung zwischen Dichtlippe und eingeführtem Schaft und andererseits einen hinreichend starren bzw. steifen bzw. unelastischen Stützbereich, um eine vollständige oder weitgehende Dichtwirkung auch dann zu gewährleisten, wenn ein in der Dichteinrichtung angeordneter Schaft in einer Richtung orthogonal zu seiner Längsachse relativ zur Dichteinrichtung verschoben wird. Eine hinreichende Steifheit des Stützbereichs hat in diesem Fall zur Folge, dass der komplette ringartige Stützbereich verschoben wird, sobald der Schaft an einer Seite die Dichtlippe vollständig verformt hat und am Stützbereich anliegt. Durch die resultierende vollständige Verschiebung des Stützbereichs kann sichergestellt werden, dass die Dichtlippe auch an der gegenüberliegenden Seite noch ganz am Schaft anliegt und ihre Dichtwirkung vollständig erfüllen kann. Dies gilt insbesondere, wenn gleichzeitig die beschriebenen Bedingungen für die Differenzen der Umfänge oder Durchmesser oder für den arithmetischen Mittelwert der Umfänge oder Durchmesser gelten.

Bei einer Dichteinrichtung, wie sie hier beschrieben ist, kann eine Verschiebung des Stützbereichs relativ zum Befestigungsbereich, beispielsweise aufgrund einer Elastizität einer den Stützbereich mit dem Befestigungsbereich verbindenden rohrförmigen Wand der Dichteinrichtung, möglich sein. Alternativ kann die Dichteinrichtung zur Verbindung mit einem Tubus oder einer anderen medizinischen Vorrichtung, die eine Verschiebung der gesamten Dichteinrichtung ermöglicht, vorgesehen und ausgebildet sein.

Bei einer Dichteinrichtung, wie sie hier beschrieben ist, ist insbesondere eine von zwei voneinander abgewandten Oberflächen der Dichtlippe bündig mit einer von zwei voneinander abgewandten Oberflächen des Stützbereichs.

Insbesondere sind die proximalen bzw. von einem Tubus oder einer anderen medizinischen Vorrichtung, mit der die Dichteinrichtung zu verbinden ist, abgewandten Oberflächen von Dichtlippe und Stützbereich bündig. Beispielsweise liegen beide proximale Oberflächen in einer Ebene oder auf einer Kugeloberfläche oder einer Oberfläche eines anderen Rotationsellipsoids. Alternativ können die distalen bzw. einem Tubus oder einer anderen medizinischen Vorrichtung, mit der die Dichteinrichtung zu verbinden ist, zugewandten Oberflächen von Dichtlippe und Stützbereich in einer Ebene, auf einer Kugeloberfläche oder einer Oberfläche eines anderen Rotationsellipsoids liegen oder sind auf andere Weise bündig sein.

Die bündige Ausgestaltung von Oberflächen der Dichtlippe und des Stützbereichs kann ihre Fertigung, insbesondere die Gestalt eines verwendeten Gusswerkzeugs vereinfachen. Eine konische oder andere trichterförmige Ausgestaltung eines Übergangsbereichs zwischen den proximalen Oberflächen des Stützbereichs und der Dichtlippe kann ein Einführen eines Schafts eines medizinischen Instruments in die Dichteinrichtung vereinfachen.

Bei einer Dichteinrichtung, wie sie hier beschrieben ist, weist die Dichtlippe im mechanisch spannungsfreien Zustand insbesondere die Gestalt eines Ausschnitts aus einer Ebene, aus einer Oberfläche eines Kreiskegels oder eines elliptischen Kegels, aus einer Oberfläche einer Pyramide, aus einer Oberfläche eines Rotationsparaboloids oder eines anderen Paraboloids oder aus einer Oberfläche einer Kugel oder eines anderen Rotationsellipsoids oder eines anderen Ellipsoids auf.

Die Dichtlippe befindet sich insbesondere dann im mechanisch spannungsfreien Zustand, wenn kein Schaft eines medizinischen Instruments in die Dichteinrichtung eingeführt ist. Die Gestalt der Dichtlippe im mechanisch spannungsfreien Zustand ist insbesondere die Gestalt der Dichtlippe nach der Fertigung der Dichteinrichtung und vor ihrer Verwendung. Die Dichtlippe weist insbesondere die Gestalt eines ringartigen bzw. zweifach zusammenhängenden Ausschnitts aus einer Ebene oder einer Oberfläche eines Kreiskegels, eines elliptischen Kegels, einer Pyramide, eines Paraboloids oder Ellipsoids auf. Sofern die Dichtlippe die Gestalt eines Ausschnitts aus einer Oberfläche einer Pyramide aufweist, weist die Grundfläche der Pyramide insbesondere einen glatten (im mathematischen Sinne differenzierbaren) oder polygonalen Rand auf.

Bei einer Dichteinrichtung, wie sie hier beschrieben ist, ist insbesondere zumindest ein Teil der Oberfläche der Dichtlippe reibungsmindernd beschichtet oder gestaltet.

Eine reibungsmindernde Beschichtung der Dichteinrichtung umfasst insbesondere Poly (p-Xylylene), das auch unter dem Markennamen Parylene vertrieben wird.

Eine reibungsmindernde Beschichtung oder Gestaltung der Dichtlippe kann die zum Bewegen eines medizinischen Instruments in der Dichteinrichtung zur Überwindung der Haftreibung und/oder der Gleitreibung erforderlichen Kräfte herabsetzen und darüber hinaus bei einer Umkehr der Bewegungsrichtung ein Umklappen der Dichtlippen verhindern. Ferner kann eine reibungsmindernde Beschichtung oder Gestaltung der Oberfläche ein allmähliches Umklappen der Dichtlippe bei einer Änderung der Richtung der Bewegung eines Schafts in der Dichteinrichtung unterstützen. Eine durch die Beschichtung verminderte Reibung kann ferner das Risiko einer Beschädigung oder Zerstörung der Dichteinrichtung, beispielsweise beim Einführen eines spitzen oder scharfkantigen medizinischen Instruments, vermindern.

Ein Tubus weist eine Dichteinrichtung, wie sie hier beschrieben ist, auf.

Der Tubus ist insbesondere der Tubus eines Trokars.

### Kurzbeschreibung der Figuren

Nachfolgend sind Ausführungsformen anhand der beigefügten Figuren näher erläutert. Es zeigen:
Figur 1 eine schematische Darstellung eines Schnitts durch eine Dichteinrichtung;
Figur 2 eine weitere schematische Darstellung eines Schnitts durch die Dichteinrichtung aus Figur 1;
Figur 3 eine weitere schematische Darstellung eines Schnitts durch die Dichteinrichtung aus Figuren 1 und 2;
Figur 4 eine weitere schematische Darstellung eines Schnitts durch die Dichteinrichtung aus Figuren 1 bis 3;
Figur 5 eine schematische Darstellung eines Schnitts durch eine weitere Dichteinrichtung;
Figur 6 eine schematische Darstellung eines Schnitts durch eine weitere Dichteinrichtung;
Figur 7 eine schematische Darstellung eines Schnitts durch eine weitere Dichteinrichtung;
Figur 8 eine schematische Darstellung eines Schnitts durch eine weitere Dichteinrichtung;

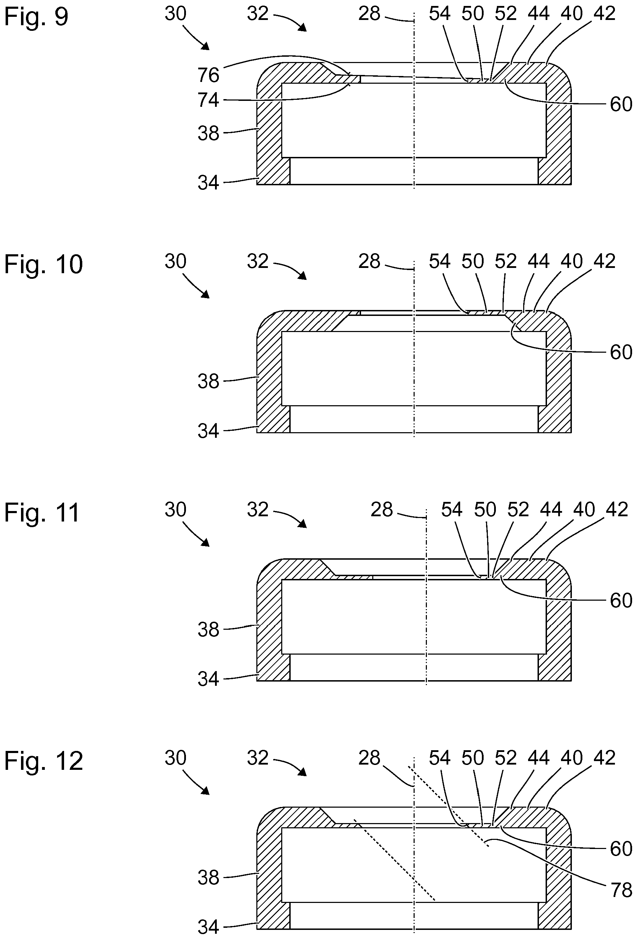

Figur 9 eine schematische Darstellung eines Schnitts durch eine weitere Dichteinrichtung;
Figur 10 eine schematische Darstellung eines Schnitts durch eine weitere Dichteinrichtung;
Figur 11 eine schematische Darstellung eines Schnitts durch eine weitere Dichteinrichtung;
Figur 12 eine schematische Darstellung eines Schnitts durch eine weitere Dichteinrichtung;

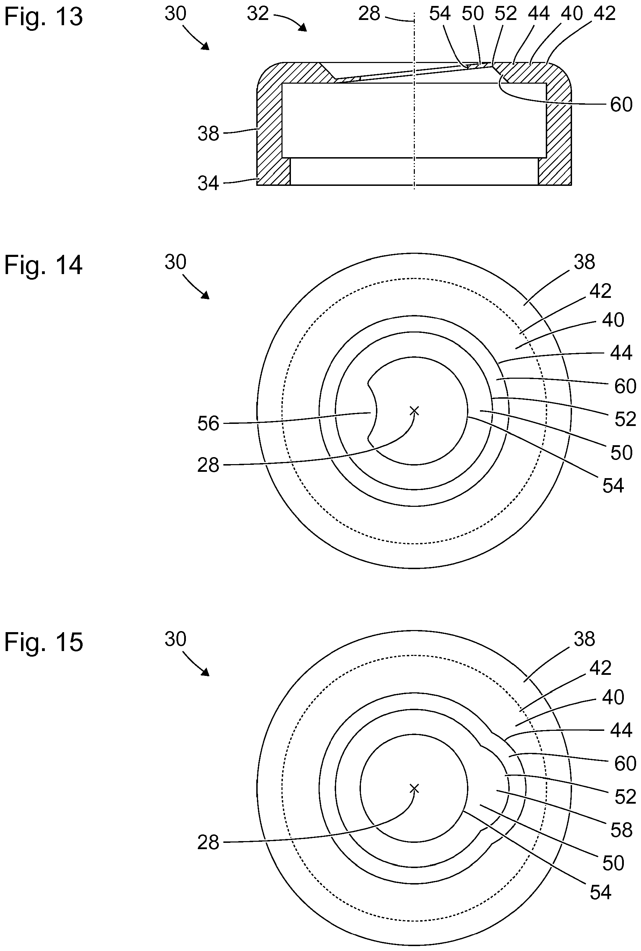

Figur 13 eine schematische Darstellung eines Schnitts durch eine weitere Dichteinrichtung;
Figur 14 eine schematische Draufsicht auf eine weitere Dichteinrichtung;
Figur 15 eine schematische Draufsicht auf eine weitere Dichteinrichtung;

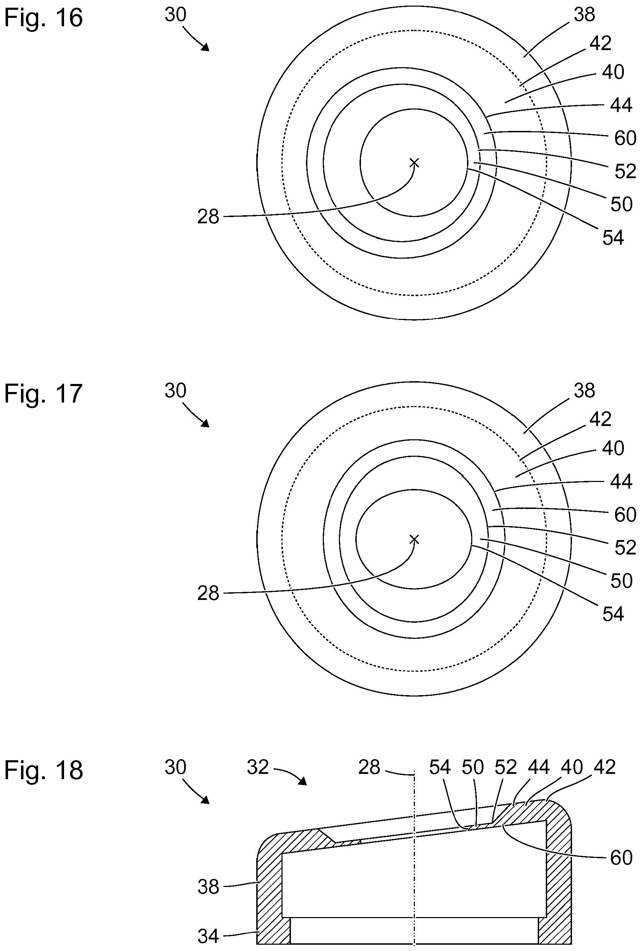

Figur 16 eine schematische Draufsicht auf eine weitere Dichteinrichtung;
Figur 17 eine schematische Draufsicht auf eine weitere Dichteinrichtung;
Figur 18 eine schematische Darstellung eines Schnitts durch eine weitere Dichteinrichtung.

### Beschreibung der Ausführungsformen

Figur 1 zeigt eine schematische Darstellung eines Schnitts durch eine Dichteinrichtung 30 zur Abdichtung einer Durchführung für einen Schaft eines medizinischen Instruments. Die Schnittebene der Figur 1 enthält eine Symmetrieachse, zu der die Dichteinrichtung 30 rotationssymmetrisch ist.

Die Dichteinrichtung 30 ist zur mechanischen Verbindung mit einem Tubus 10 eines Trokars oder mit einer anderen medizinischen Vorrichtung ausgebildet. Der Tubus 10 ist nicht Bestandteil der Dichteinrichtung 30 und deshalb in Figur 1 lediglich transparent und mit gestrichelten Konturen angedeutet. Die Dichteinrichtung 30 weist eine proximale bzw. vom Trokar 10 und bei der Verwendung von einem Patienten abgewandte Seite 32 und einen distalen bzw. dem Trokar 10 zugewandten Rand 34 auf. Am oder nahe dem distalen Rand 34 ist ein nach innen ragender Kragen 36 zum Eingriff in eine korrespondierende Nut an dem Trokar 10 vorgesehen. Damit bildet der nach innen ragende Kragen 36 einen Befestigungsbereich der Dichteinrichtung 30. Eine zylindrische Wand 38 verbindet den distalen Rand 34 mit dem nach innen ragenden Kragen 36 einerseits mit der proximalen Seite 32 der Dichteinrichtung 30 andererseits.

Die proximale Seite 32 der Dichteinrichtung 30 wird durch einen ringartigen Stützbereich 40 und eine Dichtlippe 50 gebildet. Der Stützbereich 40 und die Dichtlippe 50 weisen jeweils die Gestalt eines kreisringförmigen Ausschnitts aus einer Platte auf. Der äußere Rand 42 des Stützbereichs 40 geht in die zylindrische Wand 38 der Dichteinrichtung 30 über. Der innere Rand 44 des Stützbereichs 40 ist durch einen Übergangsbereich 60 mit dem äußeren Rand 52 der Dichtlippe 50 verbunden. Der innere Rand 54 der Dichtlippe 50 ist zur Anlage an einen in die Dichteinrichtung 30 einzuführenden Schaft eines medizinischen Instruments vorgesehen.

Bei dem dargestellten Beispiel sind die distalen bzw. dem Tubus 10 zugewandten und in Figur 1 jeweils nach unten orientierten Oberflächen des Stützbereichs 40 und der Dichtlippe 50 bündig und liegen insbesondere in einer Ebene. Die proximalen bzw. vom Tubus 10 abgewandten und in Figur 1 nach oben orientierten Oberflächen des Stützbereichs 40 und der Dichtlippe 50 liegen in zwei voneinander beabstandeten Ebenen. Der Übergangsbereich 60 weist deshalb eine konische proximale Oberfläche auf.

Die Dichteinrichtung 30 ist insbesondere einstückig als gleichzeitig mit allen beschriebenen Merkmalen hergestelltes Gussteil ausgebildet. Die Dichteinrichtung 30 weist beispielsweise ein Silikonelastomer oder ein anderes Elastomer oder ein anderes elastisches Material auf. Das Material der Dichteinrichtung 30 weist insbesondere eine Härte von ca. 40 Shore-A auf. Bei der erwähnten Rotationssymmetrie der Dichteinrichtung 30 sind der äußere Rand 42 und der innere Rand 44 des Stützbereichs 40 und der äußere Rand 52 und der innere Rand 54 der Dichtlippe 50 jeweils kreisförmig, wobei alle Mittelpunkte auf der Symmetrieachse der Dichteinrichtung 30 liegen.

Der innere Rand 54 der Dichtlippe 50 weist einen Durchmesser D₁ auf. Der innere Rand 44 des Stützbereichs 40 weist einen Durchmesser D₂ auf. Die Dichteinrichtung 30 ist für die Verwendung mit einem Schaft eines medizinischen Instruments vorgesehen und ausgebildet, der einen Durchmesser D_{S} aufweist. Die Durchmesser D₁ und D₂ der inneren Ränder 54, 44 der Dichtlippe 50 und des Stützbereichs 40 sind so gewählt, dass D₂ - D_{S} ≤ D_{S} - D₁ ist. Anders ausgedrückt ist der arithmetische Mittelwert (D₁ + D₂)/2 nicht größer als der mittlere Durchmesser D_{S} eines Schafts eines medizinischen Instruments, für den die Dichteinrichtung 30 vorgesehen und ausgebildet ist. Eine ähnliche (bei kreisförmigen Rändern 54, 44 und Querschnitt äquivalente) Bedingung lautet U₂ - U_{S} ≤ U_{S} - U₁, wobei U₁ der Umfang des inneren Rands 54 der Dichtlippe 50, U₂ der Umfang des inneren Rands 44 des Stützbereichs 40 und U_{S} der Umfang eines Schafts eines medizinischen Instruments, für den die Dichteinrichtung vorgesehen und ausgebildet ist, sind. Diese Bedingung ist mathematisch identisch mit der Bedingung, dass der arithmetische Mittelwert (U₁ + U₂)/2 des Umfangs U₁ des inneren Rands 54 der Dichtlippe 50 und des Umfangs U₂ des inneren Rands 44 des Stützbereichs 40 nicht größer ist als der Umfang U_{S} des Schafts, (U₁ + U₂)/2 ≤ U_{S}.

Die genannten Relationen zwischen den Durchmessern oder Umfängen gewährleisten, wie anhand der Figuren 2 bis 4 dargestellt ist, jederzeit eine vollständige Dichtwirkung.

Der Durchmesser D₁ des inneren Rands 54 der Dichtlippe 50 beträgt zwischen 60 % und 90 % des Durchmessers D_{S} eines Schafts eines medizinischen Instruments, für den die Dichteinrichtung vorgesehen und ausgebildet ist, 0,6 D_{S} ≤ D₁ ≤ 0,9 D_{S}. Auch dies trägt zur Dichtwirkung bei.

Die Dichtlippe 50 weist eine Dicke t₁ auf. Der Stützbereich 40 weist eine Dicke t₂ auf. Die zylindrische Wand 38 weist eine Dicke t₃ auf. Die Dicke t₁ der Dichtlippe 50 beträgt höchstens ein Fünftel der Dicke t₂ des Stützbereichs 40, t₁/t₂ ≤ 1/5. Mit diesem Dickenverhältnis ist gewährleistet, dass die Dichtlippe 50 eine wesentlich größere Elastizität aufweist als der Stützbereich 40. Dies trägt, wie anhand der Figuren 2 bis 4 dargestellt, ebenso dazu bei, dass die Dichtwirkung der Dichteinrichtung 30 auch unter widrigen Umständen erhalten bleibt. Beispielsweise betragen die Dicke t₁ der Dichtlippe 50 ca. 0,3 mm und die Dicke t₂ des Stützbereichs 40 ca. 1,5 mm.

Figur 2 zeigt eine weitere schematische Schnittdarstellung der Dichteinrichtung 30 aus Figur 1. Die Art der Darstellung, insbesondere die dargestellte Schnittebene, entspricht derjenigen der Figur 1. In Unterschied zur Figur 1 ist die Dichteinrichtung 30 ohne Tubus dargestellt. In Unterschied zur Figur 1 ist in Figur 2 ein Schaft 20 eines medizinischen Instruments in der Dichteinrichtung 30 dargestellt. Der Schaft 20 weist eine Längsachse 28 auf, die bei der dargestellten Situation mit der Symmetrieachse der Dichteinrichtung 30 zusammenfällt und in der dargestellten Schnittebene liegt.

Der innere Rand 54 der Dichtlippe 50 liegt an der äußeren Oberfläche des Schafts 20 in einem den Schaft 20 ringförmig umschließenden Bereich an. Dadurch entfaltet die Dichtlippe 50 die vorgesehene Dichtwirkung. Da der Durchmesser D_{S} des Schafts 20 größer ist als der Durchmesser D₁ des inneren Rands 54 der Dichtlippe 50 in ihrem mechanisch spannungsfreien Zustand (vgl. Figur 1), ist die Dichtlippe 50 verformt. Die resultierende mechanische Spannung innerhalb der Dichtlippe 50 bewirkt einen Anpressdruck der Dichtlippe 50, insbesondere ihres inneren Rands 54, an der äußeren Oberfläche des Schafts 20. Dieser Anpressdruck fördert die Dichtwirkung, erzeugt aber auch Gleit- und Haftreibung zwischen der Dichtlippe 50 und dem Schaft 20. Aufgrund der geringen Dicke t₁ der Dichtlippe 50 sind der Anpressdruck der Dichtlippe 50 an der äußeren Oberfläche des Schafts 20 sowie die Reibung zwischen diesen verhältnismäßig gering.

Die in Figur 2 dargestellte Verformung der Dichtlippe 50 resultiert insbesondere beim Einführen des Schafts 20 von proximal nach distal bzw. in Figur 2 von oben nach unten. Nach einer Umkehr der Bewegungsrichtung des Schafts 20 relativ zur Dichteinrichtung 30 und einer Bewegung des Schafts 20 relativ zur Dichteinrichtung 30 nach proximal kann die Dichtlippe 50 umgelegt werden, wonach der innere Rand 54 der Dichtlippe 50 nicht mehr nach distal, sondern nach proximal orientiert ist. Dieses Umlegen wird vom medizinischen Personal als Umschnappen wahrgenommen und kann eine unwillkürliche oder nicht genau dem Willen des medizinischen Personals entsprechende Bewegung des Schafts 20 bewirken. Dies kann eine Verletzungsgefahr für einen Patienten darstellen. Durch die geringe Dicke der Dichtlippe 50 sind die beim Umlegen der Dichtlippe 50 auftretenden Kräfte verhältnismäßig gering.

Figur 3 zeigt eine weitere schematische Darstellung eines Schnitts durch die Dichteinrichtung 30 aus den Figuren 1 und 2. Die Art der Darstellung, insbesondere die Schnittebene, entspricht derjenigen der Figur 1 und vor allem derjenigen der Figur 2.

In Unterschied zu Figur 2 zeigt Figur 3 eine Situation, bei der der Schaft 20 relativ zur Dichteinrichtung 30 in einer Richtung orthogonal zur Längsachse 28 des Schafts 20 und zur Symmetrieachse der Dichteinrichtung 30 verschoben ist. Der Schaft 20 ist so weit verschoben, wie es bei ausschließlicher Verformung der Dichtlippe 50 und ohne wesentliche Verformung des Stützbereichs 40 maximal möglich ist. Aufgrund der beschriebenen Relation zwischen den Durchmessern D₁, D₂ und D_{S} der inneren Ränder 54, 44 der Dichtlippe 50 und des Stützbereichs 40 sowie des Schafts 20 liegt die Dichtlippe 50 auch an der der maximalen Verformung gegenüberliegenden, in Figur 3 links dargestellten Seite noch an der äußeren Oberfläche des Schafts 20 an. Damit ist weiterhin eine vollständige Dichtwirkung gewährleistet.

Figur 4 zeigt eine weitere schematische Darstellung eines Schnitts durch die Dichteinrichtung 30 aus den Figuren 1 bis 3. Die Art der Darstellung, insbesondere die Schnittebene, entspricht derjenigen der Figur 1 und vor allem der Figuren 2 und 3.

In Figur 4 ist eine Situation gezeigt, in der der Schaft 20 relativ zur Dichteinrichtung 30 noch weiter als bei der in Figur 3 gezeigten Situation in Richtung orthogonal zur Längsachse 28 des Schafts 20 verschoben ist. Dabei wird bei dem in Figur 4 dargestellten Beispiel die zylindrische Wand 38 verformt. Alternativ oder zusätzlich könnte der in Figur 1 angedeutete, in Figur 4 nicht gezeigte Tubus 10 verformt werden. Die große Dicke t₂ des Stützbereichs 40 und seine resultierende geringe, mechanische Elastizität bewirken, dass der Stützbereich 40 nicht nur am Ort maximaler Verformung der Dichtlippe 50 (in Figur 4: rechts), sondern auch an der gegenüberliegenden Seite (in Figur 4: links) im gleichen oder fast gleichen Maße verschoben wird. Die Dichtlippe 50 liegt deshalb nicht nur am Ort maximaler Verformung, sondern auch an der gegenüberliegenden Seite noch vollständig an der äußeren Oberfläche des Schafts 20 an und entfaltet ihre vollständige Dichtwirkung.

Die anhand der Figuren 2 bis 4 dargestellte Dichtwirkung der Dichteinrichtung 30 auch bei deutlicher Verschiebung des Schafts 20 liegt insbesondere dann vor, wenn gilt t₁/t₂ ≤ 1/5, weil dann die Dichtlippe 50 hinreichend elastisch und der Stützbereich 40 hinreichend unelastisch ist.

Figur 5 zeigt eine schematische Darstellung eines Schnitts durch eine weitere Dichteinrichtung 30, die in einigen Merkmalen, Eigenschaften und Funktionen der anhand der Figuren 1 bis 4 dargestellten Dichteinrichtung ähnelt. Die Art der Darstellung, insbesondere die Schnittebene, entspricht derjenigen der Figuren 2 bis 4 und besonders der Figur 1, wobei weder ein Tubus noch ein Schaft eines medizinischen Instruments angedeutet sind. Nachfolgend sind insbesondere Merkmale und Eigenschaften beschrieben, in denen die in Figur 5 gezeigte Dichteinrichtung 30 sich von der anhand der Figuren 1 bis 4 dargestellten Dichteinrichtung unterscheidet.

Bei der in Figur 5 gezeigten Dichteinrichtung ist die Dichtlippe 50 relativ zum Stützbereich 40 weiter proximal (in den Figuren: weiter oben) angeordnet. Insbesondere ist die proximale Oberfläche der Dichtlippe 50 bündig mit der proximalen Oberfläche des Stützbereichs 40 angeordnet. Die proximalen Oberflächen der Dichtlippe 50, des Stützbereichs 40 und des Übergangsbereichs 60 liegen somit in einer Ebene. Der Übergangsbereich 60 weist eine konische distale Oberfläche auf.

Figur 6 zeigt eine schematische Darstellung eines Schnitts durch eine weitere Dichteinrichtung 30, die in einigen Merkmalen, Eigenschaften und Funktionen den anhand der Figuren 1 bis 5 dargestellten Dichteinrichtungen ähnelt. Die Art der Darstellung entspricht derjenigen der Figuren 1 bis 4 und besonders der Figur 5. Nachfolgend sind insbesondere Merkmale, Eigenschaften und Funktionen dargestellt, in denen die in Figur 6 gezeigte Dichteinrichtung sich von den anhand der Figuren 1 bis 5 dargestellten Dichteinrichtungen unterscheidet.

Bei der in Figur 6 dargestellten Dichteinrichtung ist die Dichtlippe 50 relativ zum Stützbereich 40 so angeordnet, dass weder die proximalen Oberflächen noch die distalen Oberflächen der Dichtlippe 50 und des Stützbereichs 40 bündig sind oder in einer Ebene liegen. Stattdessen weist der Übergangsbereich 60 sowohl proximal als auch distal konische Oberflächen auf.

Figur 7 zeigt eine schematische Darstellung eines Schnitts durch eine weitere Dichteinrichtung 30, die in einigen Merkmalen, Eigenschaften und Funktionen den anhand der Figuren 1 bis 6 dargestellten Dichteinrichtungen ähnelt. Die Art der Darstellung entspricht derjenigen der Figuren 1 bis 4 und besonders der Figuren 5 und 6. Nachfolgend sind insbesondere Merkmale, Eigenschaften und Funktionen dargestellt, in denen die in Figur 7 gezeigte Dichteinrichtung sich von den anhand der Figuren 1 bis 6 dargestellten Dichteinrichtungen unterscheidet.

Die in Figur 7 dargestellte Dichteinrichtung unterscheidet sich insbesondere von der in Figur 1 dargestellten Dichteinrichtung insbesondere dadurch, dass die proximale Oberfläche des Übergangsbereichs 60 zwischen Dichtlippe 50 und Stützbereich 40 nicht konisch, sondern allgemeiner trichterförmig, jedoch mit einem abgerundeten Übergang zum äußeren Rand 52 der Dichtlippe 50 ausgebildet ist. Der innere Rand 44 des Stützbereichs 40 ist durch eine scharfe Kante zum Übergangsbereich 60 definiert. Alternativ oder zusätzlich kann am inneren Rand 44 des Stützbereichs 40 ein weicher Übergang zum Übergangsbereich 60 vorgesehen sein. Entsprechend können auch die konischen distalen Oberflächen des Übergangsbereichs 60 bei den anhand der Figuren 5 und 60 dargestellten Ausführungsbeispielen abgerundet ausgebildet sein.

Figur 8 zeigt eine schematische Darstellung eines Schnitts durch eine weitere Dichteinrichtung 30, die in einigen Merkmalen, Eigenschaften und Funktionen den anhand der Figuren 1 bis 7 dargestellten Dichteinrichtungen ähnelt. Die Art der Darstellung entspricht derjenigen der Figuren 1 bis 4 und besonders der Figuren 5 bis 7. Nachfolgend sind insbesondere Merkmale, Eigenschaften und Funktionen dargestellt, in denen die in Figur 8 gezeigte Dichteinrichtung sich von den anhand der Figuren 1 bis 7 dargestellten Dichteinrichtungen unterscheidet.

Die in Figur 8 dargestellte Dichteinrichtung 30 unterscheidet sich von den anhand der Figuren 1 bis 7 dargestellten Dichteinrichtungen insbesondere dadurch, dass die Dichtlippe 50 die Gestalt eines ringförmigen Ausschnitts aus einem Konus bzw. Kreiskegel 72, der in Figur 8 in gestrichelten Linien angedeutet ist, aufweist. Die Spitze des Kreiskegels 72 zeigt nach distal, um ein Einführen eines Schafts eines medizinischen Instruments in die Dichteinrichtung durch eine zentrierende Wirkung zu vereinfachen.

Bei dem dargestellten Beispiel ist der äußere Rand 52 der Dichtlippe 50 zwischen zwei durch die proximale und die distale Oberfläche des Stützbereichs 40 definierten Ebenen angeordnet. Deshalb weist sowohl die proximale als auch die distale Oberfläche des Übergangsbereichs 60 jeweils eine konische Gestalt auf, ähnlich wie bei der anhand der Figur 6 dargestellten Dichteinrichtung.

Die proximale Oberfläche der Dichtlippe 50 ist bündig mit der proximalen Oberfläche des Übergangsbereichs 60. Die proximalen Oberflächen der Dichtlippe 50 und des Übergangsbereichs 60 schließen den gleichen Winkel mit der Längsache 28 eines in der vorgesehenen mittleren Position und Orientierung in die Dichteinrichtung 30 eingeführten Schafts ein.

Figur 9 zeigt eine schematische Darstellung eines Schnitts durch eine weitere Dichteinrichtung 30, die in einigen Merkmalen, Eigenschaften und Funktionen den anhand der Figuren 1 bis 8 dargestellten Dichteinrichtungen ähnelt. Die Art der Darstellung entspricht derjenigen der Figuren 1 bis 4 und besonders der Figuren 5 bis 8. Nachfolgend sind insbesondere Merkmale, Eigenschaften und Funktionen dargestellt, in denen die in Figur 9 gezeigte Dichteinrichtung sich von den anhand der Figuren 1 bis 8 dargestellten Dichteinrichtungen unterscheidet.

Die in Figur 9 gezeigte Dichteinrichtung 30 unterscheidet sich von den anhand der Figuren 1 bis 8 dargestellten Dichteinrichtungen insbesondere dadurch, dass die Dichtlippe 50 keine konstante Dicke aufweist. Vielmehr variiert die Dicke der Dichtlippe 50 in Richtung ihres Umfangs. Bei dem dargestellten Beispiel sind die proximale und die distale Oberfläche der Dichtlippe 50 jeweils eben, wobei die Flächennormalen einen Winkel von ca. 1,5 Grad einschließen. Da die in Figur 9 dargestellte Schnittebene so gewählt ist, dass die Flächennormalen beider Oberflächen der Dichtlippe 50 in der Schnittebene liegen, sind in Figur 9 (rechts) die minimale Dicke und (links) die maximale Dicke der Dichtlippe 50 sichtbar.

Figur 10 zeigt eine schematische Darstellung eines Schnitts durch eine weitere Dichteinrichtung 30, die in einigen Merkmalen, Eigenschaften und Funktionen den anhand der Figuren 1 bis 9 dargestellten Dichteinrichtungen ähnelt. Die Art der Darstellung entspricht derjenigen der Figuren 1 bis 4 und besonders der Figuren 5 bis 9. Nachfolgend sind insbesondere Merkmale, Eigenschaften und Funktionen dargestellt, in denen die in Figur 10 gezeigte Dichteinrichtung sich von den anhand der Figuren 1 bis 9 dargestellten Dichteinrichtungen unterscheidet.

Die in Figur 10 dargestellte Dichteinrichtung 30 unterscheidet sich von den anhand der Figuren 1 bis 9 dargestellten Dichteinrichtungen insbesondere dadurch, dass die Dichtlippe 50 eine entlang ihres Umfangs variierende Breite aufweist. Beispielsweise sind sowohl der äußere Rand 52 als auch der innere Rand 54 der Dichtlippe 50 jeweils kreisförmig, wobei die Mittelpunkte beider Ränder 52, 54 voneinander beabstandet sind. Bei dem dargestellten Beispiel liegt der Mittelpunkt des inneren Rands 54 der Dichtlippe 50 auf der Längsachse 28 eines in der vorgesehenen mittleren Position und Orientierung in die Dichteinrichtung 30 eingeführten Schafts.

Figur 11 zeigt eine schematische Darstellung eines Schnitts durch eine weitere Dichteinrichtung 30, die in einigen Merkmalen, Eigenschaften und Funktionen den anhand der Figuren 1 bis 10 dargestellten Dichteinrichtungen ähnelt. Die Art der Darstellung entspricht derjenigen der Figuren 1 bis 4 und besonders der Figuren 5 bis 10. Nachfolgend sind Merkmale, Eigenschaften und Funktionen dargestellt, in denen die in Figur 11 gezeigte Dichteinrichtung sich von den anhand der Figuren 1 bis 10 dargestellten Dichteinrichtungen unterscheidet.

Die in Figur 11 gezeigte Dichteinrichtung 30 unterscheidet sich von der anhand der Figur 10 dargestellten Dichteinrichtung insbesondere dadurch, dass die Dichtlippe 50 nicht wie bei den anhand der Figuren 5 und 10 dargestellten Dichteinrichtungen bündig mit der proximalen Oberfläche des Stützbereichs 40, sondern ähnlich wie bei den anhand der Figuren 1 bis 4, 7 und 9 dargestellten Dichteinrichtungen bündig mit der distalen Oberfläche des Stützbereichs 40 angeordnet ist. Ferner unterscheidet die in Figur 11 gezeigte Dichteinrichtung 30 sich von der anhand der Figur 10 dargestellten dadurch, dass der innere Rand 54 der Dichtlippe 50 exzentrisch positioniert ist. Beispielsweise sind der innere Rand 44 des Stützbereichs 40, der äußere Rand 52 und der innere Rand 54 der Dichtlippe 50 jeweils kreisförmig, wobei die Mittelpunkte des inneren Rands 44 des Stützbereichs 40 und des äußeren Rands 52 der Dichtlippe 50 auf einer in Figur 11 nicht gezeigten Symmetrieachse der Dichteinrichtung 30 liegen. Der Mittelpunkt des inneren Rands 54 der Dichtlippe 50 und damit die Längsachse 28 eines in der vorgesehenen mittleren Position und Orientierung in die Dichteinrichtung 30 eingeführten Schafts sind von der Symmetrieachse der Dichteinrichtung 30 beabstandet.

Figur 12 zeigt eine schematische Darstellung eines Schnitts durch eine weitere Dichteinrichtung 30, die in einigen Merkmalen, Eigenschaften und Funktionen den anhand der Figuren 1 bis 11 dargestellten Dichteinrichtungen ähnelt. Die Art der Darstellung entspricht derjenigen der Figuren 1 bis 4 und besonders der Figuren 5 bis 11. Nachfolgend sind insbesondere Merkmale, Eigenschaften und Funktionen dargestellt, in denen die in Figur 12 gezeigte Dichteinrichtung sich von den anhand der Figuren 1 bis 11 dargestellten Dichteinrichtungen unterscheidet.

Die in Figur 12 gezeigte Dichteinrichtung 30 unterscheidet sich von den anhand der Figuren 1 bis 11 dargestellten Dichteinrichtungen insbesondere in der Ausgestaltung des inneren Rands 54 der Dichtlippe 50. Bei den anhand der Figuren 1 bis 11 dargestellten Ausführungsbeispielen ist der innere Rand 54 jeweils mit der Gestalt eines schmalen ringförmigen Ausschnitts aus einer Mantelfläche eines Kreiszylinders angedeutet, wobei die Symmetrieachse des Kreiszylinders mit der Längsachse 28 eines in der vorgesehen mittleren Position und Orientierung in die Dichteinrichtung 30 eingeführten Schafts zusammenfällt. Bei der in Figur 12 gezeigten Dichteinrichtung 30 weist der innere Rand 54 der Dichtlippe 50 die Gestalt eines schmalen ringförmigen Ausschnitts aus einer Mantelfläche eines in Figur 12 durch gestrichelte Linien angedeuteten Zylinders 78, dessen Symmetrieachse (d. h. Richtung der Translationsinvarianz) nicht parallel zur Längsachse 28 eines in der vorgesehenen mittleren Position und Orientierung in die Dichteinrichtung 30 eingeführten Schafts eines medizinischen Instruments ist. Vielmehr schließen die Symmetrieachse des Zylinders 78 und die Längsachse 28 einen Winkel ein, der beispielsweise im Bereich von 15 Grad bis 45 Grad liegt und insbesondere ca. 30 Grad beträgt.

Figur 13 zeigt eine schematische Darstellung eines Schnitts durch eine weitere Dichteinrichtung 30, die in einigen Merkmalen, Eigenschaften und Funktionen den anhand der Figuren 1 bis 12 dargestellten Dichteinrichtungen ähnelt. Die Art der Darstellung entspricht derjenigen der Figuren 1 bis 4 und besonders der Figuren 5 bis 12. Nachfolgend sind insbesondere Merkmale, Eigenschaften und Funktionen dargestellt, in denen die in Figur 13 gezeigte Dichteinrichtung sich von den anhand der Figuren 1 bis 12 dargestellten Dichteinrichtungen unterscheidet.

Die in Figur 13 gezeigte Dichteinrichtung 30 unterscheidet sich von den anhand der Figuren 1 bis 12 dargestellten Dichteinrichtungen insbesondere dadurch, dass die Dichtlippe 50 nicht parallel zu einer Ebene orthogonal zur Längsachse 28 eines in der vorgesehenen mittleren Position und Orientierung in die Dichteinrichtung 30 eingeführten Schafts ist. Die Dichtlippe 50 ist stattdessen gekippt angeordnet. Bei dem dargestellten Beispiel ist der äußere Rand 52 der Dichtlippe 50 an einer Seite (in Figur 13: links) mit der distalen Oberfläche des Stützbereichs 60 und an der gegenüberliegenden Seite (in Figur 13: rechts) bündig mit der proximalen Oberfläche des Stützbereichs 60.

Figur 14 zeigt eine schematische Draufsicht auf eine Dichteinrichtung 30, die in einigen Merkmalen, Eigenschaften und Funktionen den anhand der Figuren 1 bis 13 dargestellten Dichteinrichtungen ähnelt. Die Zeichenebene der Figur 14 ist orthogonal zu den Schnittebenen der Figuren 1 bis 13 und zur Längsachse 28 eines in der vorgesehenen mittleren Position und Orientierung in die Dichteinrichtung 30 eingeführten Schafts. Die Dichteinrichtung 30 ist in einer Ansicht von proximal gezeigt, so dass die proximale Seite 32 (vgl. Figuren 1 bis 13) sichtbar ist. Die Kontur der inneren Oberfläche der zylindrischen Wand 38 der Dichteinrichtung 30 ist durch eine gestrichelte Linie angedeutet.

Bei dem dargestellten Beispiel weist die zylindrische Wand 38 der Dichteinrichtung 30 die Gestalt eines Ausschnitts einer Mantelfläche eines Kreiszylinders auf, die Konturen ihres Querschnitts sind deshalb jeweils kreisförmig. Auch der innere Rand 44 des Stützbereichs 40 und der äußere Rand 52 der Dichtlippe 50 sind jeweils kreisförmig, wobei alle Kreismittelpunkte auf der Längsachse 28 eines in der vorgesehenen mittleren Position und Orientierung in die Dichteinrichtung 30 eingeführten Schafts liegen. Abweichend von dieser weitgehenden Rotationssymmetrie der Dichteinrichtung ist an der Dichtlippe 50 eine Zunge 56 vorgesehen, die in die von der Dichtlippe 50 umschlossene Öffnung für einen Schaft hineinragt. Der innere Rand 54 der Dichtlippe 50 weist deshalb eine Gestalt auf, die im Bereich der Zunge 56 von einer reinen Kreisform abweicht.

Figur 15 zeigt eine schematische Darstellung einer Draufsicht auf eine weitere Dichteinrichtung 30, die in einigen Merkmalen, Eigenschaften und Funktionen den anhand der Figuren 1 bis 14 dargestellten Dichteinrichtungen ähnelt. Die Art der Darstellung entspricht derjenigen der Figur 14. Nachfolgend sind insbesondere Merkmale, Eigenschaften und Funktionen beschrieben, in denen die in Figur 15 gezeigte Dichteinrichtung 30 sich von den anhand der Figuren 1 bis 14 dargestellten Dichteinrichtungen unterscheidet.

Bei der in Figur 15 gezeigten Dichteinrichtung 30 weist die Dichtlippe 50 einen buchtartig verbreiterten Bereich 58 auf. Der äußere Rand 52 der Dichtlippe 50 und der innere Rand 44 des Stützbereichs 40 weisen am bzw. benachbart zum buchartig verbreiterten Bereich 58 der Dichtlippe eine von einer Kreisform abweichende Gestalt auf. Der innere Rand 54 der Dichtlippe 50 ist kreisförmig.

Figur 16 zeigt eine schematische Darstellung einer Draufsicht auf eine weitere Dichteinrichtung 30, die in einigen Merkmalen, Eigenschaften und Funktionen den anhand der Figuren 1 bis 15 dargestellten Dichteinrichtungen ähnelt. Die Art der Darstellung entspricht derjenigen der Figuren 14 und 15. Nachfolgend sind Merkmale, Eigenschaften und Funktionen beschrieben, in denen die in Figur 16 gezeigte Dichteinrichtung 30 sich von den anhand der Figuren 1 bis 15 dargestellten Dichteinrichtungen unterscheidet.

Bei der in Figur 16 gezeigten Dichteinrichtung 30 weisen der innere Rand 44 des Stützbereichs 40, der äußere Rand 52 und der innere Rand 54 der Dichtlippe 50 jeweils die Gestalt eines Kreises auf, wobei die Mittelpunkte des inneren Rands 44 des Stützbereichs 40 und des äußeren Rands 52 der Dichtlippe 50 gegenüber der Symmetrieachse anderer Merkmale der Dichteinrichtung 30 und insbesondere gegenüber dem Mittelpunkt des inneren Rands 54 der Dichtlippe 50 verschoben sind. Die Dichtlippe 50 weist deshalb ähnlich wie bei den anhand der Figuren 10, 11, 14 und 15 dargestellten Dichteinrichtungen eine entlang ihres Umfangs variierende Breite auf.

Figur 17 zeigt eine schematische Darstellung einer Draufsicht auf eine weitere Dichteinrichtung 30, die in einigen Merkmalen, Eigenschaften und Funktionen den anhand der Figuren 1 bis 16 dargestellten Dichteinrichtungen ähnelt. Die Art der Darstellung entspricht derjenigen der Figuren 14 bis 16. Nachfolgend sind Merkmale, Eigenschaften und Funktionen beschrieben, in denen die in Figur 17 gezeigte Dichteinrichtung 30 sich von den anhand der Figuren 1 bis 16 dargestellten Dichteinrichtungen unterscheidet.

Bei der in Figur 17 gezeigten Dichteinrichtung 30 weisen der innere Rand 44 des Stützbereichs 40, der äußere Rand 52 und der innere Rand 54 der Dichtlippe 50 jeweils die Gestalt einer Ellipse auf, wobei die Flächenmittelpunkte der Ellipsen auf der Symmetrieachse anderer Merkmale der Dichteinrichtung 30 und der Längsachse 28 eines in der vorgesehenen mittleren Position und Orientierung in die Dichteinrichtung 30 eingeführten Schafts liegen. Bei dem dargestellten Beispiel sind die großen Hauptachsen des elliptischen inneren Rands 44 des Stützbereichs 40 und des elliptischen äußeren Rands 52 der Dichtlippe 50 parallel zur kleinen Hauptachse des elliptischen inneren Rands 54 der Dichtlippe 50 und die kleinen Hauptachsen des elliptischen inneren Rands 44 des Stützbereichs 40 und des elliptischen äußeren Rands 52 der Dichtlippe 50 parallel zur großen Hauptachse des elliptischen inneren Rands 54 der Dichtlippe 50. Die Dichtlippe 50 weist deshalb eine entlang ihres Umfangs kontinuierlich variierende Breite auf.

Figur 18 zeigt eine schematische Darstellung eines Schnitts durch eine weitere Dichteinrichtung 30, die in einigen Merkmalen, Eigenschaften und Funktionen den anhand der Figuren 1 bis 17 dargestellten Dichteinrichtungen ähnelt. Die Art der Darstellung entspricht derjenigen der Figuren 1 bis 4 und besonders der Figuren 5 bis 13. Nachfolgend sind insbesondere Merkmale, Eigenschaften und Funktionen dargestellt, in denen die in Figur 18 gezeigte Dichteinrichtung sich von den anhand der Figuren 1 bis 17 dargestellten Dichteinrichtungen unterscheidet.

Die in Figur 18 gezeigte Dichteinrichtung 30 unterscheidet sich von den anhand der Figuren 1 bis 12 dargestellten Dichteinrichtungen insbesondere dadurch, dass die Dichtlippe 50 nicht parallel zu einer Ebene orthogonal zur Längsachse 28 eines in der vorgesehenen mittleren Position und Orientierung in die Dichteinrichtung 30 eingeführten Schafts ist. Darin ähnelt die in Figur 18 gezeigte Dichteinrichtung 30 der anhand der Figur 13 dargestellten Dichteinrichtung.

In Gegensatz zu den anhand der Figuren 1 bis 12 dargestellten Dichteinrichtungen und in Gegensatz zu der anhand der Figur 13 dargestellten Dichteinrichtung ist jedoch bei der in Figur 18 gezeigten Dichteinrichtung 30 auch der Stützbereich 40 nicht in einer Ebene orthogonal zur Längsachse 28 eines in der vorgesehenen mittleren Position und Orientierung in die Dichteinrichtung 30 eingeführten Schafts angeordnet. Vielmehr sind sowohl der Stützbereich 40 als auch die Dichtlippe 50 parallel oder im Wesentlichen parallel zu einer Ebene, die gegenüber der Längsachse 28 gekippt ist, angeordnet. Bei dem dargestellten Beispiel schließt die Normale einer Ebene, zu der der Stützbereich 40 und die Dichtlippe 50 parallel oder im Wesentlichen parallel sind, mit der Längsachse 28 eines in der vorgesehenen mittleren Position und Orientierung in die Dichteinrichtung 30 eingeführten Schafts einen Winkel im Bereich von 5 Grad bis 10 Grad ein. Der Winkel kann jedoch alternativ kleiner als 5 Grad oder größer als 10 Grad sein.

Bei den anhand der Figuren 1 bis 18 dargestellten Dichteinrichtungen, insbesondere bei den anhand der Figuren 1 bis 13 und 18 dargestellten Dichteinrichtungen, sind die Stützbereiche jeweils als ringförmige Ausschnitte aus ebenen Platten ausgebildet. Die Stützbereiche weisen also jeweils einen ebenen oder im Wesentlichen ebenen proximale Oberflächenbereich und einen ebenen und im Wesentlichen ebenen distalen Oberflächenbereich auf, die parallel zu einander sind. Abweichend davon können die gesamten Stützbereiche oder die proximalen Oberflächenbereiche und/oder die distalen Oberflächenbereiche der Stützbereiche jeweils die Gestalt von ringförmigen Ausschnitten von gekrümmten Flächen aufweisen, beispielsweise von Kugeloberflächen oder von Oberfläche anderer Rotationsellipsoide.

In den Figuren 1 bis 18, insbesondere in den Figuren 1 bis 13 und 18, sind die Querschnitte jeweils als homogen angedeutet. Die dargestellten Dichteinrichtungen 30 bestehen also jeweils aus einem einzigen Material, das sowohl den Stützbereich 40 als auch die Dichtlippe 50 und insbesondere auch die zylindrische Wand 38 und den nach innen ragenden Kragen 36 (als Befestigungsbereich) bildet. Abweichend davon kann jede der dargestellten Dichteinrichtungen aus mehreren verschiedenen Materialien bestehen, wobei Bereiche aus unterschiedlichen Materialien insbesondere stoff- und/oder formschlüssig miteinander verbunden sind. Beispielsweise sind die Dichtlippe aus einem vergleichsweise weichen Material und der Stützbereich oder ein Teil des Stützbereichs oder die zylindrische Wand 38 aus einem vergleichsweise harten Material gebildet.

Bei den anhand der Figuren 9 bis 18 dargestellten Dichteinrichtungen 30 sind die Dichtlippen 50 jeweils nicht rotationssymmetrisch zur Längsachse 28 eines in der vorgesehenen mittleren Position und Orientierung in die Dichteinrichtung 30 eingeführten Schafts. Die Verformung der Dichtlippe 50 durch einen in die Dichteinrichtung 30 eingeführten Schafts - ähnlich wie anhand der Figur 2 dargestellt - bewirkt deshalb eine elastische Verformung und eine damit einhergehende mechanische Spannung innerhalb der Dichtlippe 50 sowie einen Anpressdruck der Dichtlippe 50 an der äußeren Oberfläche des Schafts, die nicht rotationssymmetrisch sind, sondern entlang des Umfangs der Dichtlippe 50 variieren. Dadurch kann bei einer Umkehr der Bewegungsrichtung eines Schafts in der Dichteinrichtung 30 ein allmähliches anstelle eines plötzlichen und gleichzeitigen Umklappens bzw. Umlegens der Dichtlippe 50 begünstigt sein. Ein allmähliches Umlegen der Dichtlippe 50 geht - unter anderem abhängig von der Reibung zwischen der Dichtlippe 50 und der äußeren Oberfläche des Schafts - beispielsweise von der Stelle aus, an der die Dichtlippe 50 die größte Breite oder die geringste Dicke aufweist und breitet sich von diesem Ort ausgehend entlang des Umfangs der Dichtlippe aus bis die Dichtlippe 50 vollständig umgelegt ist. Das allmähliche Umlegen der Dichtlippe 50 ist weniger spürbar und wird von medizinischem Personal als weniger störend empfunden.

Bei allen anhand der Figuren 1 bis 18 dargestellten Dichteinrichtungen können die gesamte Oberfläche oder zumindest ein Teil oder mehrere Teile der Oberfläche der Dichtlippe 40 reibungsmindernd beschichtet oder gestaltet sein. Beispielsweise kann die gesamte Oberfläche oder ein Teil der Oberfläche der Dichtlippe 40 mit Poly (p-Xylylene), das auch unter dem Markennamen Parylene vertrieben wird, beschichtet sein. Eine reibungsmindernde Beschichtung kann eine Haft- und/oder Gleitreibung zwischen der Dichteinrichtung 30 und einem in die Dichteinrichtung 30 eingeführten medizinischen Instrument reduzieren.

Merkmale der anhand der Figuren 1 bis 18 dargestellten Dichteinrichtungen sind teilweise miteinander kombinierbar. Beispielsweise können bei den anhand der Figuren 9 bis 18 dargestellten Dichteinrichtungen die Dichtlippen und die Übergangsbereiche ähnliche wie bei den anhand der Figuren 5 bis 8 dargestellten Dichteinrichtungen angeordnet und ausgebildet sein. Ferner kann eine asymmetrische Ausgestaltung der Dichtlippe 50 ähnlich wie anhand der Figuren 9 bis 18 dargestellt auch dann vorliegen, wenn Dichtlippe 50 und Stützbereich 40 nicht klar von einander unterscheidbar sind, sondern beispielsweise mit einem gemeinsamen keilförmigen Querschnitt kontinuierlich in einander übergehen.

### Bezugszeichen

- 10: Tubus eines Trokars
- 20: medizinisches Instrument oder Schaft eines medizinischen Instruments
- 28: Längsachse des Schafts 20
- 30: Dichteinrichtung
- 32: proximale Seite der Dichteinrichtung 30
- 34: distaler Rand der Dichteinrichtung 30
- 36: nach innen ragender Kragen am distalen Rand 34 der Dichteinrichtung 30
- 38: zylindrische Wand der Dichteinrichtung 30 zwischen distalem Rand 34 und Stützbereich 40
- 40: Stützbereich der Dichteinrichtung 30
- 42: äußerer Rand des Stützbereichs 40
- 44: innerer Rand des Stützbereichs 40
- 50: Dichtlippe der Dichteinrichtung 30
- 52: äußerer Rand der Dichtlippe 50
- 54: innerer Rand der Dichtlippe 50
- 56: Zunge der Dichtlippe 50
- 58: buchtartig verbreiterter Bereich der Dichtlippe 50
- 60: Übergangsbereich zwischen Stützbereich 40 und Dichtlippe 50
- 72: Kegel
- 74: innere bzw. distale Oberfläche der Dichtlippe 50
- 76: äußere bzw. proximale Oberfläche der Dichtlippe 50
- 78: Zylinder

## Patentansprüche

1. **Dichteinrichtung** (30) zur Abdichtung einer Durchführung für einen Schaft (20) eines medizinischen Instruments, mit:
einem **Befestigungsbereich** (36) zur Befestigung der Dichteinrichtung (30) an einem Tubus (10) oder einer anderen medizinischen Vorrichtung;
einer ringartigen **Dichtlippe** (50) mit einem inneren Rand (54), der vorgesehen und ausgebildet ist, um an einer äußeren Oberfläche eines in die Dichteinrichtung (30) eingeführten Schafts (20) fluiddicht anzuliegen,
wobei die Dichtlippe (50) **nicht rotationssymmetrisch** zur Längsachse (28) eines in der vorgesehenen mittleren Position und Orientierung in die Dichteinrichtung (30) eingeführten Schafts (20) ist,
wobei im mechanisch spannungsfreien Zustand der Dichtlippe (50) der **Flächenmittelpunkt** der durch den inneren Rand (54) der Dichtlippe (50) begrenzten Fläche vom **Flächenmittelpunkt** der durch den äußeren Rand (52) der Dichtlippe (50) begrenzten Fläche **beabstandet** ist.

2. Dichteinrichtung (30) nach gemäß dem vorangehenden Anspruch, bei der
die Dichtlippe (50) im mechanisch spannungsfreien Zustand eine in Richtung ihres Umfangs **variierende Dicke** aufweist.

3. Dichteinrichtung (30) nach dem vorangehenden Anspruch, bei der
**Flächennormalen** zweier voneinander abgewandter **Oberflächen** der Dichtlippe (50) an einander gegenüber liegenden Orten an den Oberflächen im mechanisch spannungsfreien Zustand der Dichtlippe (50) **nicht parallel** sind.

4. Dichteinrichtung (30) nach einem der vorangehenden Ansprüche, bei der
der innere Rand (54) der Dichtlippe (50) die Gestalt eines **Ausschnitts einer Mantelfläche eines Zylinders,** dessen Symmetrieachse nicht parallel zur mittleren Flächennormale zweier voneinander abgewandter Oberflächen (74, 76) der Dichtlippe (50) ist, aufweist.

5. Dichteinrichtung (30) nach einem der vorangehenden Ansprüche, bei der
die **mittlere Flächennormale** der Dichtlippe (50) **nicht parallel** zur Längsachse (28) eines in der vorgesehenen mittleren Position und Orientierung in die Dichteinrichtung (30) eingeführten Schafts (20) ist.

6. Dichteinrichtung (30) nach einem der vorangehenden Ansprüche, bei der
der Umfang des inneren Rands (54) der Dichtlippe (50) mindestens **sechs Zehntel** und höchstens **neun Zehntel** des Umfangs eines Schafts (20) eines medizinischen Instruments, für den die Dichteinrichtung (30) vorgesehen und ausgebildet ist, beträgt.

7. Dichteinrichtung (30) nach einem der vorangehenden Ansprüche, bei der
die mittlere **Dicke** (t₁) der Dichtlippe (50) **höchstens ein Fünftel** der mittleren Dicke (t₂) des Stützbereichs (40) beträgt.

8. Dichteinrichtung (30) nach einem der vorangehenden Ansprüche, bei der die Dichtlippe (50) im mechanisch spannungsfreien Zustand eine in Richtung ihres Umfangs kontinuierlich variierenden Dicke aufweist.

9. **Tubus** (10) mit einer Dichteinrichtung (30) nach einem der vorangehenden Ansprüche.

## Claims

1. Sealing device (30) for sealing a feedthrough for a shaft (20) of a medical instrument, comprising:
a fastening region (36) for fastening the sealing device (30) to a tube (10) or another medical device;
an annular sealing lip (50) with an inner edge (54) which is provided and designed to bear in a fluid-tight manner on an outer surface of a shaft (20) inserted into the sealing device (30),
wherein the sealing lip (50) is non-rotationally symmetrical to the longitudinal axis (28) of a shaft (20) inserted in the intended central position and orientation into the sealing device (30), wherein, in the mechanically tension-free state of the sealing lip (50), the centroid of the area limited by the inner edge (54) of the sealing lip (50) is spaced apart from the centroid limited by the outer edge (52) of the sealing lip (50).

2. Sealing device (30) according to the preceding claim, in which
the sealing lip (50) in the mechanically tension-free state has a thickness varying in the direction of its circumference.

3. Sealing device (30) according to the preceding claim, in which
surface normals of two surfaces of the sealing lip (50) facing away from each other at mutually opposite locations on the surfaces are not parallel in the mechanically tension-free state of the sealing lip (50).

4. Sealing device (30) according to one of the preceding claims, in which
the inner edge (54) of the sealing lip (50) has the shape of a cutout of a lateral face of a cylinder, of which the axis of symmetry is not parallel to the average surface normal of two surfaces (74, 76) of the sealing lip (50) facing away from each other.

5. Sealing device (30) according to one of the preceding claims, in which
the average surface normal of the sealing lip (50) is not parallel to the longitudinal axis (28) of a shaft (20) inserted in the intended central position and orientation into the sealing device (30).

6. Sealing device (30) according to one of the preceding claims, in which
the circumference of the inner edge (54) of the sealing lip (50) is at least six tenths and at most nine tenths of the circumference of a shaft (20) of the medical instrument for which the sealing device (30) is provided and designed.

7. Sealing device (30) according to one of the preceding claims, in which
the average thickness (t₁) of the sealing lip (50) is at most a fifth of the average thickness (t₂) of the support region (40).

8. Sealing device (30) according to one of the preceding claims, in which the sealing lip (50) in the mechanically tension-free state has a thickness continuously varying in the direction of its circumference.

9. Tube (10) having a sealing device (30) according to one of the preceding claims.

## Revendications

1. Dispositif d'étanchéité (30) pour étanchéifier un passage pour une tige (20) d'un instrument médical, comprenant :
une zone de fixation (36) pour fixer le dispositif d'étanchéité (30) à un tube (10) ou à un autre appareil médical ;
une lèvre d'étanchéité annulaire (50) ayant un bord intérieur (54), qui est prévu et configuré pour s'appuyer de manière étanche aux fluides contre une surface extérieure d'une tige (20) insérée dans le dispositif d'étanchéité (30),
la lèvre d'étanchéité (50) ne présentant pas de symétrie de rotation par rapport à l'axe longitudinal (28) d'une tige (20) insérée dans le dispositif d'étanchéité (30) dans la position et l'orientation centrales prévues,
dans l'état mécaniquement libre de tension de la lèvre d'étanchéité (50), le centroïde de la surface délimitée par le bord intérieur (54) de la lèvre d'étanchéité (50) étant espacé du centroïde de la surface délimitée par le bord extérieur (52) de la lèvre d'étanchéité (50) .

2. Dispositif d'étanchéité (30) selon la revendication précédente, dans lequel
la lèvre d'étanchéité (50), à l'état mécaniquement libre de tension, présente une épaisseur variable dans la direction de sa circonférence.

3. Dispositif d'étanchéité (30) selon la revendication précédente, dans lequel
des normales à la surface de deux surfaces détournées l'une de l'autre de la lèvre d'étanchéité (50) ne sont pas parallèles à des emplacements opposés l'un à l'autre sur les surfaces à l'état mécaniquement libre de tension de la lèvre d'étanchéité (50).

4. Dispositif d'étanchéité (30) selon l'une quelconque des revendications précédentes, dans lequel le bord intérieur (54) de la lèvre d'étanchéité (50) présente la forme d'une découpe d'une surface d'enveloppe d'un cylindre, dont l'axe de symétrie n'est pas parallèle à la normale à la surface centrale de deux surfaces (74, 76) détournées l'une de l'autre de la lèvre d'étanchéité (50).

5. Dispositif d'étanchéité (30) selon l'une quelconque des revendications précédentes, dans lequel la normale à la surface centrale de la lèvre d'étanchéité (50) n'est pas parallèle à l'axe longitudinal (28) d'une tige (20) insérée dans le dispositif d'étanchéité (30) dans la position et l'orientation centrales prévues.

6. Dispositif d'étanchéité (30) selon l'une quelconque des revendications précédentes, dans lequel la circonférence du bord intérieur (54) de la lèvre d'étanchéité (50) représente au moins six dixièmes et au plus neuf dixièmes de la circonférence d'une tige (20) d'un instrument médical pour lequel le dispositif d'étanchéité (30) est prévu et configuré.

7. Dispositif d'étanchéité (30) selon l'une quelconque des revendications précédentes, dans lequel l'épaisseur moyenne (t₁) de la lèvre d'étanchéité (50) est d'au plus un cinquième de l'épaisseur moyenne (t₂) de la zone de support (40).

8. Dispositif d'étanchéité (30) selon l'une quelconque des revendications précédentes, dans lequel la lèvre d'étanchéité (50) présente, à l'état mécaniquement libre de tension, une épaisseur variable en continu dans la direction de sa circonférence.

9. Tube (10) comprenant un dispositif d'étanchéité (30) selon l'une quelconque des revendications précédentes.
